# EUROPEAN PATENT APPLICATION

(11) **EP 2 692 870 A1**
(43) Date of publication of application: **05.02.2014**
(21) Application number: 12179283.2
(22) Date of filing: 03.08.2012
(51) Int. Cl.: C12Q 1/68

(54) **Method for nucleic acid amplification**

(71) Applicant: Alacris Theranostics GmbH, 14195 Berlin (DE)
(72) Inventor: Glökler, Jörn, 10783 Berlin (DE)
(74) Representative: Fanelli Haag Kilger PLLC

(57) **Abstract**

The present invention relates to a method for nucleic acid amplification, and detection or sequencing, comprising the steps of,
a. providing a double stranded target nucleic acid molecule for amplification,
b. terminally attaching to said target nucleic acid molecule both up-stream and downstream a first and second linker nucleic acid comprising a protelomerase target sequence,
c. reacting said target nucleic acid molecule with a protelomerase, preferably as encoded by SEQ ID NO. 1, such that the target nucleic acid is converted to a circular target nucleic acid,
d. amplifying the circular target nucleic acid in an amplification reaction comprising a polymerase and at least one first primer which binds outside the protelomerase target sequence such that an in parts self complimentary amplification product is synthesized.

SEQUENCE LISTING

<110>  Alacris Theranostics GmbH

<120>  Method of nucleic acid amplification

<130>  B106-0021EP1

<160>  17

<170>  PatentIn version 3.5

<210>  1
<211>  1719
<212>  DNA
<213>  Bacillus bacteriophage phi29 polymerase

<400>  1
atgccgagaa agatgtatag ttgtgacttt gagacaacta ctaaagtgga agactgtagg      60

gtatgggcgt atggttatat gaatatagaa gatcacagtg agtacaaaat aggtaatagc     120

ctggatgagt ttatggcgtg ggtgttgaag gtacaagctg atctatattt ccataacctc     180

aaatttgacg gagcttttat cattaactgg ttggaacgta atggttttaa gtggtcggct     240

gacggattgc caaacacata taatacgatc atatctcgca tgggacaatg gtacatgatt     300

gatatatgtt taggctacaa agggaaacgt aagatacata cagtgatata tgacagctta     360

aagaaactac cgtttcctgt taagaagata gctaaagact ttaaactaac tgttcttaaa     420

ggtgatattg attaccacaa agaaagacca gtcggctata agataacacc cgaagaatac     480

gcctatatta aaaacgatat tcagattatt gcggaagctc tgttaattca gtttaagcaa     540

ggtttagacc ggatgacagc aggcagtgac agtctaaaag gtttcaagga tattataacc     600

actaagaaat tcaaaaaggt gtttcctaca ttgagtcttg gactcgataa ggaagtgaga     660

tacgcctata gaggtggttt tacatggtta aatgataggt tcaaagaaaa agaaatcgga     720

gaaggcatgg tcttcgatgt taatagtcta tatcctgcac agatgtatag ccgtctcctt     780

ccatatggtg aacctatagt attcgagggt aaatacgttt gggacgaaga ttacccacta     840

cacatacagc atatcagatg tgagttcgaa ttgaaagagg gctatatacc cactatacag     900

ataaaaagaa gtaggtttta taaaggtaat gagtacctaa aaagtagcgg cggggagata     960

gccgacctct ggttgtcaaa tgtagaccta gaattaatga aagaacacta cgatttatat    1020

aacgttgaat atatcagcgg cttaaaattt aaagcaacta caggtttgtt taaagatttt    1080

atagataaat ggacgtacat caagacgaca tcagaaggag cgatcaagca actagcaaaa    1140

ctgatgttaa acagtctata cggtaaattc gctagtaacc ctgatgttac agggaaagtc    1200

ccttatttaa aagagaatgg ggcgctaggt ttcagacttg gagaagagga aacaaaagac    1260

cctgtttata cacctatggg cgttttcatc actgcatggg ctagatacac gacaattaca    1320

gcggcacagg cttgttatga tcggataata tactgtgata ctgacagcat acatttaacg    1380

ggtacagaga tacctgatgt aataaaagat atagttgacc ctaagaaatt gggatactgg    1440

gcacatgaaa gtacattcaa aagagctaaa tatctgagac agaagaccta tatacaagac 1500

atctatatga aagaagtaga tggtaagtta gtagaaggta gtccagatga ttacactgat 1560

ataaaattta gtgttaaatg tgcgggaatg actgacaaga ttaagaaaga ggttacgttt 1620

gagaatttca aagtcggatt cagtcggaaa atgaagccta agcctgtgca agtgccgggc 1680

ggggtggttc tggttgatga cacattcaca atcaaataa 1719

<210> 2
<211> 572
<212> PRT
<213> Bacillus bacteriophage phi29 DNA polymerase

<400> 2

Met Pro Arg Lys Met Tyr Ser Cys Asp Phe Glu Thr Thr Thr Lys Val
1               5                   10                  15

Glu Asp Cys Arg Val Trp Ala Tyr Gly Tyr Met Asn Ile Glu Asp His
            20                  25                  30

Ser Glu Tyr Lys Ile Gly Asn Ser Leu Asp Glu Phe Met Ala Trp Val
        35                  40                  45

Leu Lys Val Gln Ala Asp Leu Tyr Phe His Asn Leu Lys Phe Asp Gly
    50                  55                  60

Ala Phe Ile Ile Asn Trp Leu Glu Arg Asn Gly Phe Lys Trp Ser Ala
65                  70                  75                  80

Asp Gly Leu Pro Asn Thr Tyr Asn Thr Ile Ile Ser Arg Met Gly Gln
                85                  90                  95

Trp Tyr Met Ile Asp Ile Cys Leu Gly Tyr Lys Gly Lys Arg Lys Ile
            100                 105                 110

His Thr Val Ile Tyr Asp Ser Leu Lys Lys Leu Pro Phe Pro Val Lys
        115                 120                 125

Lys Ile Ala Lys Asp Phe Lys Leu Thr Val Leu Lys Gly Asp Ile Asp
    130                 135                 140

Tyr His Lys Glu Arg Pro Val Gly Tyr Lys Ile Thr Pro Glu Glu Tyr
145                 150                 155                 160

Ala Tyr Ile Lys Asn Asp Ile Gln Ile Ile Ala Glu Ala Leu Leu Ile
                165                 170                 175

Gln Phe Lys Gln Gly Leu Asp Arg Met Thr Ala Gly Ser Asp Ser Leu
            180                 185                 190

Lys Gly Phe Lys Asp Ile Ile Thr Thr Lys Lys Phe Lys Lys Val Phe
        195                 200                 205

Pro Thr Leu Ser Leu Gly Leu Asp Lys Glu Val Arg Tyr Ala Tyr Arg
    210                 215                 220

Gly Gly Phe Thr Trp Leu Asn Asp Arg Phe Lys Glu Lys Glu Ile Gly
225                 230                 235                 240

Glu Gly Met Val Phe Asp Val Asn Ser Leu Tyr Pro Ala Gln Met Tyr
                245                 250                 255

Ser Arg Leu Leu Pro Tyr Gly Glu Pro Ile Val Phe Glu Gly Lys Tyr
            260                 265                 270

Val Trp Asp Glu Asp Tyr Pro Leu His Ile Gln His Ile Arg Cys Glu
        275                 280                 285

Phe Glu Leu Lys Glu Gly Tyr Ile Pro Thr Ile Gln Ile Lys Arg Ser
    290                 295                 300

Arg Phe Tyr Lys Gly Asn Glu Tyr Leu Lys Ser Ser Gly Gly Glu Ile
305                 310                 315                 320

Ala Asp Leu Trp Leu Ser Asn Val Asp Leu Glu Leu Met Lys Glu His
                325                 330                 335

Tyr Asp Leu Tyr Asn Val Glu Tyr Ile Ser Gly Leu Lys Phe Lys Ala
            340                 345                 350

Thr Thr Gly Leu Phe Lys Asp Phe Ile Asp Lys Trp Thr Tyr Ile Lys
        355                 360                 365

Thr Thr Ser Glu Gly Ala Ile Lys Gln Leu Ala Lys Leu Met Leu Asn
    370                 375                 380

Ser Leu Tyr Gly Lys Phe Ala Ser Asn Pro Asp Val Thr Gly Lys Val
385                 390                 395                 400

Pro Tyr Leu Lys Glu Asn Gly Ala Leu Gly Phe Arg Leu Gly Glu Glu
                405                 410                 415

Glu Thr Lys Asp Pro Val Tyr Thr Pro Met Gly Val Phe Ile Thr Ala
            420                 425                 430

Trp Ala Arg Tyr Thr Thr Ile Thr Ala Ala Gln Ala Cys Tyr Asp Arg
        435                 440                 445

Ile Ile Tyr Cys Asp Thr Asp Ser Ile His Leu Thr Gly Thr Glu Ile
    450                 455                 460

Pro Asp Val Ile Lys Asp Ile Val Asp Pro Lys Lys Leu Gly Tyr Trp
465                 470                 475                 480

Ala His Glu Ser Thr Phe Lys Arg Ala Lys Tyr Leu Arg Gln Lys Thr
                485                 490                 495

Tyr Ile Gln Asp Ile Tyr Met Lys Glu Val Asp Gly Lys Leu Val Glu
            500                 505                 510

Gly Ser Pro Asp Asp Tyr Thr Asp Ile Lys Phe Ser Val Lys Cys Ala
        515                 520                 525

Gly Met Thr Asp Lys Ile Lys Lys Glu Val Thr Phe Glu Asn Phe Lys
    530                 535                 540

Val Gly Phe Ser Arg Lys Met Lys Pro Lys Pro Val Gln Val Pro Gly
545                 550                 555                 560

Gly Val Val Leu Val Asp Asp Thr Phe Thr Ile Lys
                565                 570

<210>  3
<211>  1563
<212>  DNA
<213>  Halomonas phage phiHAP-1 protelomerase

<400>  3
atgagcggtg agtcacgtag aaaggtcgat ttagcggaat tgatagagtg gttgctcagc      60

gagatcaaag agatcgacgc cgatgatgag atgccacgta aagagaaaac caagcgcatg     120

gcgcggctgg cacgtagctt caaaacgcgc ctgcatgatg acaagcgccg caaggattct     180

gagcggatcg cggtcacgac ctttcgccgc tacatgacag aagcgcgcaa ggcggtgact     240

gcgcagaact ggcgccatca cagcttcgac cagcagatcg agcggctggc cagccgctac     300

ccggcttatg ccagcaagct ggaagcgctc ggcaagctga ccgatatcag cgccattcgt     360

atggcccacc gcgagctgct cgaccagatc cgcaacgatg acgacgctta tgaggacatc     420

cgggcgatga agctggacca tgaaatcatg cgccacctga cgttgagctc tgcacagaaa     480

agcacgctgg ctgaagaggc cagcgagacg ctggaagagc gcgcggtgaa cacggtcgag     540

atcaactacc actggttgat ggagacggtt tacgagctgc tgagtaaccg ggagagaatg     600

gtcgatgggg agtatcgcgg ctttttcagt tacctagcgc ttgggctggc gctggccacc     660

gggcgtcgct cgatcgaggt gctgaagacc ggacggatca cgaaggtggg cgagtatgag     720

ctggagttca gcggccaggc gaaaaagcgc ggcggcgtcg actatagcga ggcttaccac     780

atttataccc tggtgaaagc tgacctggtg atcgaagcgt gggatgagct tcgctcgctg 840

ccggaagctg ctgagctgca gggcatggac aacagcgatg tgaaccgccg cacggcgaag 900

acgctcaaca cgctcactaa gcggatcttt aacaacgatg agcgcgtttt caaggacagc 960

cgggcgatct gggcgcggct ggtgtttgag ctgcacttct cgcgcgacaa gcgctggaag 1020

aaagtcaccg aggacgtgtt ctggcgtgag atgctggggc atgaggacat ggatacacag 1080

cgcagctacc gcgcctttaa aatcgactac gacgagccgg atcaagccga ccaggaagat 1140

tacgaacacg ctagccgcct cgccgcgctg caggcgctgg acggccatga gcagcttgag 1200

agcagcgacg cccaggcgcg tgtgcatgcc tgggtgaaag cgcagatcga gcaggagcct 1260

gacgcgaaaa ttacgcagtc tctgatcagc cgggagctgg gcgtttatcg ccctgccata 1320

aaagcgtacc tggagctggc gcgagaggcg ctcgacgcgc cgaacgtcga tctggacaag 1380

gtcgcggcgg cagtgccgaa ggaagtagcc gaggcgaagc cccggctgaa cgcccaccca 1440

caaggggatg gcaggtgggt cggggtggct tcaatcaacg gggtggaagt tgcacgggtg 1500

ggcaaccagg caggccggat cgaagcgatg aaagcggcct ataaagcggc gggtgggcgc 1560

tga 1563

<210> 4
<211> 520
<212> PRT
<213> Halomonas phage phiHAP-1 protelomerase

<400> 4

Met Ser Gly Glu Ser Arg Arg Lys Val Asp Leu Ala Glu Leu Ile Glu
1               5                   10                  15

Trp Leu Leu Ser Glu Ile Lys Glu Ile Asp Ala Asp Asp Glu Met Pro
            20                  25                  30

Arg Lys Glu Lys Thr Lys Arg Met Ala Arg Leu Ala Arg Ser Phe Lys
        35                  40                  45

Thr Arg Leu His Asp Asp Lys Arg Arg Lys Asp Ser Glu Arg Ile Ala
    50                  55                  60

Val Thr Thr Phe Arg Arg Tyr Met Thr Glu Ala Arg Lys Ala Val Thr
65                  70                  75                  80

Ala Gln Asn Trp Arg His His Ser Phe Asp Gln Gln Ile Glu Arg Leu
                85                  90                  95

Ala Ser Arg Tyr Pro Ala Tyr Ala Ser Lys Leu Glu Ala Leu Gly Lys
            100                 105                 110

Leu Thr Asp Ile Ser Ala Ile Arg Met Ala His Arg Glu Leu Leu Asp

115                 120                 125

Gln Ile Arg Asn Asp Asp Asp Ala Tyr Glu Asp Ile Arg Ala Met Lys
    130                 135                 140

Leu Asp His Glu Ile Met Arg His Leu Thr Leu Ser Ser Ala Gln Lys
145                 150                 155                 160

Ser Thr Leu Ala Glu Glu Ala Ser Glu Thr Leu Glu Glu Arg Ala Val
                165                 170                 175

Asn Thr Val Glu Ile Asn Tyr His Trp Leu Met Glu Thr Val Tyr Glu
            180                 185                 190

Leu Leu Ser Asn Arg Glu Arg Met Val Asp Gly Glu Tyr Arg Gly Phe
        195                 200                 205

Phe Ser Tyr Leu Ala Leu Gly Leu Ala Leu Ala Thr Gly Arg Arg Ser
    210                 215                 220

Ile Glu Val Leu Lys Thr Gly Arg Ile Thr Lys Val Gly Glu Tyr Glu
225                 230                 235                 240

Leu Glu Phe Ser Gly Gln Ala Lys Lys Arg Gly Gly Val Asp Tyr Ser
                245                 250                 255

Glu Ala Tyr His Ile Tyr Thr Leu Val Lys Ala Asp Leu Val Ile Glu
            260                 265                 270

Ala Trp Asp Glu Leu Arg Ser Leu Pro Glu Ala Ala Glu Leu Gln Gly
        275                 280                 285

Met Asp Asn Ser Asp Val Asn Arg Arg Thr Ala Lys Thr Leu Asn Thr
    290                 295                 300

Leu Thr Lys Arg Ile Phe Asn Asn Asp Glu Arg Val Phe Lys Asp Ser
305                 310                 315                 320

Arg Ala Ile Trp Ala Arg Leu Val Phe Glu Leu His Phe Ser Arg Asp
                325                 330                 335

Lys Arg Trp Lys Lys Val Thr Glu Asp Val Phe Trp Arg Glu Met Leu
            340                 345                 350

Gly His Glu Asp Met Asp Thr Gln Arg Ser Tyr Arg Ala Phe Lys Ile
        355                 360                 365

Asp Tyr Asp Glu Pro Asp Gln Ala Asp Gln Glu Asp Tyr Glu His Ala
    370                 375                 380

Ser Arg Leu Ala Ala Leu Gln Ala Leu Asp Gly His Glu Gln Leu Glu
385                 390                 395                 400

Ser Ser Asp Ala Gln Ala Arg Val His Ala Trp Val Lys Ala Gln Ile
                405                 410                 415

Glu Gln Glu Pro Asp Ala Lys Ile Thr Gln Ser Leu Ile Ser Arg Glu
            420                 425                 430

Leu Gly Val Tyr Arg Pro Ala Ile Lys Ala Tyr Leu Glu Leu Ala Arg
        435                 440                 445

Glu Ala Leu Asp Ala Pro Asn Val Asp Leu Asp Lys Val Ala Ala Ala
    450                 455                 460

Val Pro Lys Glu Val Ala Glu Ala Lys Pro Arg Leu Asn Ala His Pro
465                 470                 475                 480

Gln Gly Asp Gly Arg Trp Val Gly Val Ala Ser Ile Asn Gly Val Glu
                485                 490                 495

Val Ala Arg Val Gly Asn Gln Ala Gly Arg Ile Glu Ala Met Lys Ala
            500                 505                 510

Ala Tyr Lys Ala Ala Gly Gly Arg
        515                 520

<210>  5
<211>  1887
<212>  DNA
<213>  Yersinia phage PY54 protelomerase

<400>  5
atgggttact tattattttt atttggaggc gttatgaaaa tccattttcg cgatttagtt      60

agtggtttag ttaaagagat cgatgaaata gaaaaatcag accgggcgca gggtgacaaa     120

actcggcgtt atcagggcgc ggccagaaag ttcaaaaatg ccgtgtttat ggataaacgg     180

aaatatcgcg gtaacggtat gaagaataga atatcgttaa caacatttaa taaatattta     240

agtcgagcac gttctcggtt tgaagaaagg cttcaccata gttttcctca atctatagca     300

actatctcaa ataaatatcc tgcattcagc gaaataataa aagatctgga taatagaccc     360

gctcatgaag ttagaataaa acttaaagaa ttaataactc atcttgaatc cggtgttaat     420

ttattagaaa aaataggtag cttagggaaa ataaaaccat ctacagctaa aaaaatagtt     480

agcttaaaaa aaatgtaccc atcatgggct aatgatctag atactttaat tagtactgaa     540

gatgctacag aattacaaca aaagttagag caagggaccg acctacttaa cgcattacat     600

tctctaaaag taaaccatga agttatgtat gcattaacga tgcagccttc tgacagagct     660

gcattaaaag ctaggcatga cgctgccctt cactttaaaa agcgtaacat cgtacctatc 720

gattatcccg gctatatgca acgaatgacg gacatactac atcttccaga tatagctttt 780

gaagattcga tggcatcact tgccccttta gcatttgctc tagcagctgc tagcggtcgc 840

agacaaattg aaatactaat tactggtgag tttgacgcca aaaataaaag catcattaaa 900

ttttctggac aagcaaaaaa aagaatggcc gtttcaggtg gacattatga aatatacagt 960

ctaattgact cagagctatt cattcaacgg ttagagtttt tacgttctca tagctcaata 1020

cttcgattac aaaatttgga aatagcacat gatgaacatc gtactgaact atctgttatt 1080

aacggttttg tagccaaacc tttaaatgat gcagcaaaac agttctttgt cgatgacaga 1140

agagtattta aagatacccg tgcaatttac gctcgcatag catatgaaaa atggtttaga 1200

acagatcctc gctgggcgaa gtgcgacgaa gatgttttct tctctgaatt attaggccat 1260

gacgacccag atactcagct ggcatataaa caattcaagc tggtaaattt caatccaaaa 1320

tggacaccta atatatcaga tgaaaaccct cggttagctg cacttcaaga gcttgacaat 1380

gatatgcccg gcctagcacg tggcgatgcg gcagttcgca tacatgagtg ggttaaagag 1440

caactggcgc agaaccctgc ggcaaaaata actgcatacc aaatcaagaa aaatttaaat 1500

tgtcgaaatg acttggccag ccgatacatg gcatggtgtg ctgacgcgct aggggttgtt 1560

attggtgatg atggacaggc aaggccagaa gaactcccac catcgctcgt gcttgatatt 1620

aacgctgatg acactgacgc tgaagaagat gaaatagagg aagactttac tgatgaggaa 1680

atagacgaca ccgaattcga cgtatcagat aacgccagtg atgaagataa gcccgaagat 1740

aaacctcgct ttgcagcacc aattcgtaga agtgaggact cttggctgat taaatttgaa 1800

tttgctggca agcaatatag ctgggagggt aatgccgaaa gtgttatcga tgcgatgaaa 1860

caagcatgga ctgaaaatat ggagtaa 1887

<210> 6
<211> 628
<212> PRT
<213> Yersinia phage PY54

<400> 6

Met Gly Tyr Leu Leu Phe Leu Phe Gly Gly Val Met Lys Ile His Phe
1               5                   10                  15

Arg Asp Leu Val Ser Gly Leu Val Lys Glu Ile Asp Glu Ile Glu Lys
            20                  25                  30

Ser Asp Arg Ala Gln Gly Asp Lys Thr Arg Arg Tyr Gln Gly Ala Ala
        35                  40                  45

Arg Lys Phe Lys Asn Ala Val Phe Met Asp Lys Arg Lys Tyr Arg Gly
    50                  55                  60

Asn Gly Met Lys Asn Arg Ile Ser Leu Thr Thr Phe Asn Lys Tyr Leu
65                  70                  75                  80

Ser Arg Ala Arg Ser Arg Phe Glu Glu Arg Leu His His Ser Phe Pro
                85                  90                  95

Gln Ser Ile Ala Thr Ile Ser Asn Lys Tyr Pro Ala Phe Ser Glu Ile
            100                 105                 110

Ile Lys Asp Leu Asp Asn Arg Pro Ala His Glu Val Arg Ile Lys Leu
        115                 120                 125

Lys Glu Leu Ile Thr His Leu Glu Ser Gly Val Asn Leu Leu Glu Lys
    130                 135                 140

Ile Gly Ser Leu Gly Lys Ile Lys Pro Ser Thr Ala Lys Lys Ile Val
145                 150                 155                 160

Ser Leu Lys Lys Met Tyr Pro Ser Trp Ala Asn Asp Leu Asp Thr Leu
                165                 170                 175

Ile Ser Thr Glu Asp Ala Thr Glu Leu Gln Gln Lys Leu Glu Gln Gly
            180                 185                 190

Thr Asp Leu Leu Asn Ala Leu His Ser Leu Lys Val Asn His Glu Val
        195                 200                 205

Met Tyr Ala Leu Thr Met Gln Pro Ser Asp Arg Ala Ala Leu Lys Ala
    210                 215                 220

Arg His Asp Ala Ala Leu His Phe Lys Lys Arg Asn Ile Val Pro Ile
225                 230                 235                 240

Asp Tyr Pro Gly Tyr Met Gln Arg Met Thr Asp Ile Leu His Leu Pro
                245                 250                 255

Asp Ile Ala Phe Glu Asp Ser Met Ala Ser Leu Ala Pro Leu Ala Phe
            260                 265                 270

Ala Leu Ala Ala Ala Ser Gly Arg Arg Gln Ile Glu Ile Leu Ile Thr
        275                 280                 285

Gly Glu Phe Asp Ala Lys Asn Lys Ser Ile Ile Lys Phe Ser Gly Gln
    290                 295                 300

Ala Lys Lys Arg Met Ala Val Ser Gly Gly His Tyr Glu Ile Tyr Ser
305                 310                 315                 320

Leu Ile Asp Ser Glu Leu Phe Ile Gln Arg Leu Glu Phe Leu Arg Ser
                325                 330                 335

His Ser Ser Ile Leu Arg Leu Gln Asn Leu Glu Ile Ala His Asp Glu
            340                 345                 350

His Arg Thr Glu Leu Ser Val Ile Asn Gly Phe Val Ala Lys Pro Leu
        355                 360                 365

Asn Asp Ala Ala Lys Gln Phe Phe Val Asp Asp Arg Arg Val Phe Lys
    370                 375                 380

Asp Thr Arg Ala Ile Tyr Ala Arg Ile Ala Tyr Glu Lys Trp Phe Arg
385                 390                 395                 400

Thr Asp Pro Arg Trp Ala Lys Cys Asp Glu Asp Val Phe Phe Ser Glu
                405                 410                 415

Leu Leu Gly His Asp Asp Pro Asp Thr Gln Leu Ala Tyr Lys Gln Phe
            420                 425                 430

Lys Leu Val Asn Phe Asn Pro Lys Trp Thr Pro Asn Ile Ser Asp Glu
        435                 440                 445

Asn Pro Arg Leu Ala Ala Leu Gln Glu Leu Asp Asn Asp Met Pro Gly
    450                 455                 460

Leu Ala Arg Gly Asp Ala Ala Val Arg Ile His Glu Trp Val Lys Glu
465                 470                 475                 480

Gln Leu Ala Gln Asn Pro Ala Ala Lys Ile Thr Ala Tyr Gln Ile Lys
                485                 490                 495

Lys Asn Leu Asn Cys Arg Asn Asp Leu Ala Ser Arg Tyr Met Ala Trp
            500                 505                 510

Cys Ala Asp Ala Leu Gly Val Val Ile Gly Asp Asp Gly Gln Ala Arg
        515                 520                 525

Pro Glu Glu Leu Pro Pro Ser Leu Val Leu Asp Ile Asn Ala Asp Asp
    530                 535                 540

Thr Asp Ala Glu Glu Asp Glu Ile Glu Glu Asp Phe Thr Asp Glu Glu
545                 550                 555                 560

Ile Asp Asp Thr Glu Phe Asp Val Ser Asp Asn Ala Ser Asp Glu Asp
                565                 570                 575

Lys Pro Glu Asp Lys Pro Arg Phe Ala Ala Pro Ile Arg Arg Ser Glu
            580                 585                 590

Asp Ser Trp Leu Ile Lys Phe Glu Phe Ala Gly Lys Gln Tyr Ser Trp
        595                 600                 605

Glu Gly Asn Ala Glu Ser Val Ile Asp Ala Met Lys Gln Ala Trp Thr
    610                 615                 620

Glu Asn Met Glu
625

<210>  7
<211>  1923
<212>  DNA
<213>  Klebsiella phage phiK02 protelomerase

<400>  7
atgcgtaagg tgaaaattgg tgagctaatc aattcgcttg tgagcgaggt cgaggcaatc      60

gatgcctctg atcgtccgca aggcgataaa acgaagaaaa ttaaagccgc agcattaaaa     120

tataagaatg cattatttaa tgacaaaaga aagtttcgcg gtaaaggttt agaaaaaaga     180

atttctgcca acacgttcaa ctcgtatatg agtcgggcaa ggaaaagatt tgatgataga     240

ttgcatcata actttgaaaa gaatgtaatt aaactatcag aaaaatatcc tttatatagt     300

gaagaattat cttcgtggct ttctatgcct gcggcatcaa ttagacagca tatgtcaaga     360

ttgcaagcca agctaaaaga gataatgcca ttggcagaag acttatccaa tataaagatt     420

ggtacaaaaa atagcgaagc aaaaataaat aaactcgcta ataaatatcc tgaatggcaa     480

ttcgctatta gtgatttaaa tagcgaagat tggaaggata aaagagatta tctttataaa     540

ctattccaac aaggttcttc gctcctggaa gacttgaata acctgaaagt aaaccatgag     600

gttctctatc atctgcagct tagttctgcc gagcgaacct ctatccagca gcgctgggcc     660

aacgtcctca gcgagaaaaa gcgcaacgtt gtcgtgattg actatccgcg ctatatgcag     720

gccatctacg atataatcaa caagcctata gtttcgttcg atttgactac tcgtcgtggt     780

atggccccgc tggcgttcgc ccttgccgcg ctatctggtc gccgaatgat tgaaatcatg     840

ctccagggtg aattttccgt cgcaggtaaa tatacagtaa cattcctggg gcaagctaaa     900

aaacgctcgg aagataaagg tatatcaagg aaaatatata ccttatgcga cgctacttta     960

tttgttagtt tggtaaatga acttcgctca tgccccgctg ctgcggattt tgatgaagta    1020

ataaaaggat atggcgaaaa tgacactcgc tcagaaaatg ggcgtattaa tgcaattctc    1080

gctacagctt ttaatccgtg ggtaaaaact ttcttaggcg atgaccgccg cgtttataaa    1140

gatagccgcg ctatttacgc ccgtattgcc tatgaaatgt tcttccgcgt tgaccctcgg    1200

tggaagaatg ttgatgagga tgtattcttc atggagattc tcggccatga cgatgaaaac    1260

acccaactgc actataagca gtttaaattg gctaacttct ccagaacatg gcgaccaaat    1320

gtcggcgagg agaatgcccg cctagcggcg ctgcaaaagc tggatagcat gatgccagat 1380

tttgccaggg gcgacgccgg ggttcgtatt catgagaccg tgaagcagct ggtggagcag 1440

gacccatcga taaaaatcac aaacagcacc ctgcgaccgt ttaacttcag taccaggctg 1500

attcctcgct acctggagtt tgccgccgat gcattgggcc agttcgtcgg tgaaaatggg 1560

caatggcaac tgaaggatga ggcgcctgca atagtcctgc ctgatgagga aattcttgag 1620

cctatggacg acgtcgatct cgatgacgaa aaccatgatg atgaaacgct ggatgacgat 1680

gagatcgaag tggacgaaag cgaaggagag gaactggagg aagcgggcga cgctgaagag 1740

gccgaggtgg ctgaacagga agagaagcac cctggcaagc caaactttaa agcgccgagg 1800

gataatggcg atggtaccta catggtggaa tttgaattcg gtggccgtca ttacgcctgg 1860

tccggtgccg ccggtaatcg ggtagaggca atgcaatctg cctggagtgc ctacttcaag 1920

tga 1923

<210> 8
<211> 640
<212> PRT
<213> Klebsiella phage phiK02 protelomerase

<400> 8

Met Arg Lys Val Lys Ile Gly Glu Leu Ile Asn Ser Leu Val Ser Glu
1               5                   10                  15

Val Glu Ala Ile Asp Ala Ser Asp Arg Pro Gln Gly Asp Lys Thr Lys
            20                  25                  30

Lys Ile Lys Ala Ala Ala Leu Lys Tyr Lys Asn Ala Leu Phe Asn Asp
        35                  40                  45

Lys Arg Lys Phe Arg Gly Lys Gly Leu Glu Lys Arg Ile Ser Ala Asn
    50                  55                  60

Thr Phe Asn Ser Tyr Met Ser Arg Ala Arg Lys Arg Phe Asp Asp Arg
65                  70                  75                  80

Leu His His Asn Phe Glu Lys Asn Val Ile Lys Leu Ser Glu Lys Tyr
                85                  90                  95

Pro Leu Tyr Ser Glu Glu Leu Ser Ser Trp Leu Ser Met Pro Ala Ala
            100                 105                 110

Ser Ile Arg Gln His Met Ser Arg Leu Gln Ala Lys Leu Lys Glu Ile
        115                 120                 125

Met Pro Leu Ala Glu Asp Leu Ser Asn Ile Lys Ile Gly Thr Lys Asn
    130                 135                 140

Ser Glu Ala Lys Ile Asn Lys Leu Ala Asn Lys Tyr Pro Glu Trp Gln
145                 150                 155                 160

Phe Ala Ile Ser Asp Leu Asn Ser Glu Asp Trp Lys Asp Lys Arg Asp
                165                 170                 175

Tyr Leu Tyr Lys Leu Phe Gln Gln Gly Ser Ser Leu Leu Glu Asp Leu
            180                 185                 190

Asn Asn Leu Lys Val Asn His Glu Val Leu Tyr His Leu Gln Leu Ser
        195                 200                 205

Ser Ala Glu Arg Thr Ser Ile Gln Gln Arg Trp Ala Asn Val Leu Ser
    210                 215                 220

Glu Lys Lys Arg Asn Val Val Val Ile Asp Tyr Pro Arg Tyr Met Gln
225                 230                 235                 240

Ala Ile Tyr Asp Ile Ile Asn Lys Pro Ile Val Ser Phe Asp Leu Thr
                245                 250                 255

Thr Arg Arg Gly Met Ala Pro Leu Ala Phe Ala Leu Ala Ala Leu Ser
            260                 265                 270

Gly Arg Arg Met Ile Glu Ile Met Leu Gln Gly Glu Phe Ser Val Ala
        275                 280                 285

Gly Lys Tyr Thr Val Thr Phe Leu Gly Gln Ala Lys Lys Arg Ser Glu
    290                 295                 300

Asp Lys Gly Ile Ser Arg Lys Ile Tyr Thr Leu Cys Asp Ala Thr Leu
305                 310                 315                 320

Phe Val Ser Leu Val Asn Glu Leu Arg Ser Cys Pro Ala Ala Ala Asp
                325                 330                 335

Phe Asp Glu Val Ile Lys Gly Tyr Gly Glu Asn Asp Thr Arg Ser Glu
            340                 345                 350

Asn Gly Arg Ile Asn Ala Ile Leu Ala Thr Ala Phe Asn Pro Trp Val
        355                 360                 365

Lys Thr Phe Leu Gly Asp Asp Arg Arg Val Tyr Lys Asp Ser Arg Ala
    370                 375                 380

Ile Tyr Ala Arg Ile Ala Tyr Glu Met Phe Phe Arg Val Asp Pro Arg
385                 390                 395                 400

Trp Lys Asn Val Asp Glu Asp Val Phe Phe Met Glu Ile Leu Gly His
                405                 410                 415

Asp Asp Glu Asn Thr Gln Leu His Tyr Lys Gln Phe Lys Leu Ala Asn
            420                 425                 430

Phe Ser Arg Thr Trp Arg Pro Asn Val Gly Glu Glu Asn Ala Arg Leu
        435                 440                 445

Ala Ala Leu Gln Lys Leu Asp Ser Met Met Pro Asp Phe Ala Arg Gly
    450                 455                 460

Asp Ala Gly Val Arg Ile His Glu Thr Val Lys Gln Leu Val Glu Gln
465                 470                 475                 480

Asp Pro Ser Ile Lys Ile Thr Asn Ser Thr Leu Arg Pro Phe Asn Phe
                485                 490                 495

Ser Thr Arg Leu Ile Pro Arg Tyr Leu Glu Phe Ala Ala Asp Ala Leu
            500                 505                 510

Gly Gln Phe Val Gly Glu Asn Gly Gln Trp Gln Leu Lys Asp Glu Ala
        515                 520                 525

Pro Ala Ile Val Leu Pro Asp Glu Glu Ile Leu Glu Pro Met Asp Asp
    530                 535                 540

Val Asp Leu Asp Asp Glu Asn His Asp Asp Glu Thr Leu Asp Asp Asp
545                 550                 555                 560

Glu Ile Glu Val Asp Glu Ser Glu Gly Glu Glu Leu Glu Glu Ala Gly
                565                 570                 575

Asp Ala Glu Glu Ala Glu Val Ala Glu Gln Glu Glu Lys His Pro Gly
            580                 585                 590

Lys Pro Asn Phe Lys Ala Pro Arg Asp Asn Gly Asp Gly Thr Tyr Met
        595                 600                 605

Val Glu Phe Glu Phe Gly Gly Arg His Tyr Ala Trp Ser Gly Ala Ala
    610                 615                 620

Gly Asn Arg Val Glu Ala Met Gln Ser Ala Trp Ser Ala Tyr Phe Lys
625                 630                 635                 640

<210>  9
<211>  1617
<212>  DNA
<213>  Vibrio phage VP882 protelomerase

<400>  9
atgagcggcg aaagtagaca aaaggtaaac ctcgaggagt taataaatga gctcgtcgag      60

gaggtgaaaa ccatcgatga caatgaggcg attactcggt ctgaaaaaac caagttgatc     120

accagggcgg cgactaaatt caagaccaag ctgcacgacg ataagcgccg gaaggatgcg     180

accagaatcg ctctgagcac ctatcgtaag tacatgacaa tggccagggc agcagttact     240

gagcagaact ggaaacacca cagtctcgag cagcagatag agcggctggc caaaaagcac     300

ccgcaatacg ctgagcagct ggtggccatc ggggccatgg ataacatcac cgagttgcgc     360

ctggcgcatc gcgacctcct gaagagcatc aaggacaacg atgaagcctt cgaggatatc     420

cgcagcatga agttagacca cgaggtaatg cgccatctga cgctacccag tgcgcaaaag     480

gcgagactgg cagaggaagc cgccgaggcg ttgaccgaga agaaaaccgc cacggtcgac     540

atcaactatc acgagctgat ggccggcgtg gtggagctgt tgaccaagaa gaccaagacg     600

gtcggcagcg acagcaccta cagcttcagc cggctggcgc ttggtattgg cctggctacc     660

ggtcgtcgtt ctatcgagat actgaagcag ggcgagttca aaaaggtgga tgagcagcgg     720

ctcgagttct ctggccaagc gaaaaagcgc ggcggtgccg actattcaga gacctatacc     780

atttacaccc tggtcgactc cgacctggta ctgatggcgc tgaagaacct gcgagagttg     840

ccagaagttc gcgcactgga tgagtacgac caactgggcg agattaagcg gaacgacgcc     900

atcaataaac gctgtgcaaa aacgctcaac caaaccgcca agcagttctt tggcagcgac     960

gagcgcgtgt tcaaagatag tcgtgccatc tgggcgcgtc tggcttatga gttgtttttt    1020

caacgtgatc cgcgctggaa aaagaaagac gaggacgttt tctggcagga gatgctgggc    1080

cacgaggaca tcgagactca gaaagcctat aagcaattca aggtcgacta cagcgaacct    1140

gagcagccgg tgcacaagcc tggcaaattt aagagcagag ctgaagccct cgcggcgctc    1200

gactcaaatg aggacattac cacccgctca tccatggcca agatccacga ctgggtgaaa    1260

gagcgtattg cggaagaccc cgaggcgaac atcacacagt cactcatcac ccgggaactg    1320

ggctcaggcc gtaaggtgat caaggactac ctcgacctgg ctgacgatgc ccttgctgtg    1380

gtgaatactc ctgtcgatga cgcagtcgtc gaggttccag ctgatgtgcc ggcagcagaa    1440

aaacagccga agaaagcgca gaagcccaga ctcgtggctc accaggttga tgatgagcac    1500

tgggaagcct gggcgctggt ggaaggcgag gaggtggcca gggtgaaaat caagggcacc    1560

cgcgttgagg caatgacagc cgcatgggag gccagccaaa aggcactcga tgactaa       1617

<210>  10
<211>  538
<212>  PRT
<213>  Vibrio phage VP882 protelomerase

<400>  10

Met Ser Gly Glu Ser Arg Gln Lys Val Asn Leu Glu Glu Leu Ile Asn

1               5                   10                  15

Glu Leu Val Glu Glu Val Lys Thr Ile Asp Asp Asn Glu Ala Ile Thr
            20                  25                  30

Arg Ser Glu Lys Thr Lys Leu Ile Thr Arg Ala Ala Thr Lys Phe Lys
        35                  40                  45

Thr Lys Leu His Asp Asp Lys Arg Arg Lys Asp Ala Thr Arg Ile Ala
    50                  55                  60

Leu Ser Thr Tyr Arg Lys Tyr Met Thr Met Ala Arg Ala Ala Val Thr
65                  70                  75                  80

Glu Gln Asn Trp Lys His His Ser Leu Glu Gln Gln Ile Glu Arg Leu
                85                  90                  95

Ala Lys Lys His Pro Gln Tyr Ala Glu Gln Leu Val Ala Ile Gly Ala
            100                 105                 110

Met Asp Asn Ile Thr Glu Leu Arg Leu Ala His Arg Asp Leu Leu Lys
        115                 120                 125

Ser Ile Lys Asp Asn Asp Glu Ala Phe Glu Asp Ile Arg Ser Met Lys
    130                 135                 140

Leu Asp His Glu Val Met Arg His Leu Thr Leu Pro Ser Ala Gln Lys
145                 150                 155                 160

Ala Arg Leu Ala Glu Glu Ala Ala Glu Ala Leu Thr Glu Lys Lys Thr
                165                 170                 175

Ala Thr Val Asp Ile Asn Tyr His Glu Leu Met Ala Gly Val Val Glu
            180                 185                 190

Leu Leu Thr Lys Lys Thr Lys Thr Val Gly Ser Asp Ser Thr Tyr Ser
        195                 200                 205

Phe Ser Arg Leu Ala Leu Gly Ile Gly Leu Ala Thr Gly Arg Arg Ser
    210                 215                 220

Ile Glu Ile Leu Lys Gln Gly Glu Phe Lys Lys Val Asp Glu Gln Arg
225                 230                 235                 240

Leu Glu Phe Ser Gly Gln Ala Lys Lys Arg Gly Gly Ala Asp Tyr Ser
                245                 250                 255

Glu Thr Tyr Thr Ile Tyr Thr Leu Val Asp Ser Asp Leu Val Leu Met
            260                 265                 270

Ala Leu Lys Asn Leu Arg Glu Leu Pro Glu Val Arg Ala Leu Asp Glu
        275                 280                 285

Tyr Asp Gln Leu Gly Glu Ile Lys Arg Asn Asp Ala Ile Asn Lys Arg
    290                 295                 300

Cys Ala Lys Thr Leu Asn Gln Thr Ala Lys Gln Phe Phe Gly Ser Asp
305                 310                 315                 320

Glu Arg Val Phe Lys Asp Ser Arg Ala Ile Trp Ala Arg Leu Ala Tyr
                325                 330                 335

Glu Leu Phe Phe Gln Arg Asp Pro Arg Trp Lys Lys Lys Asp Glu Asp
            340                 345                 350

Val Phe Trp Gln Glu Met Leu Gly His Glu Asp Ile Glu Thr Gln Lys
        355                 360                 365

Ala Tyr Lys Gln Phe Lys Val Asp Tyr Ser Glu Pro Glu Gln Pro Val
    370                 375                 380

His Lys Pro Gly Lys Phe Lys Ser Arg Ala Glu Ala Leu Ala Ala Leu
385                 390                 395                 400

Asp Ser Asn Glu Asp Ile Thr Thr Arg Ser Ser Met Ala Lys Ile His
                405                 410                 415

Asp Trp Val Lys Glu Arg Ile Ala Glu Asp Pro Glu Ala Asn Ile Thr
            420                 425                 430

Gln Ser Leu Ile Thr Arg Glu Leu Gly Ser Gly Arg Lys Val Ile Lys
        435                 440                 445

Asp Tyr Leu Asp Leu Ala Asp Asp Ala Leu Ala Val Val Asn Thr Pro
    450                 455                 460

Val Asp Asp Ala Val Val Glu Val Pro Ala Asp Val Pro Ala Ala Glu
465                 470                 475                 480

Lys Gln Pro Lys Lys Ala Gln Lys Pro Arg Leu Val Ala His Gln Val
                485                 490                 495

Asp Asp Glu His Trp Glu Ala Trp Ala Leu Val Glu Gly Glu Glu Val
            500                 505                 510

Ala Arg Val Lys Ile Lys Gly Thr Arg Val Glu Ala Met Thr Ala Ala
        515                 520                 525

Trp Glu Ala Ser Gln Lys Ala Leu Asp Asp
    530                 535

<210>  11
<211>  1896
<212>  DNA
<213>  Escherichia coli bacteriophage N15 protelomerase

<400>  11
atgagcaagg taaaaatcgg tgagttgatc aacacgcttg tgaatgaggt agaggcaatt      60

gatgcctcag accgcccaca aggcgacaaa acgaagagaa ttaaagccgc agccgcacgg     120

tataagaacg cgttatttaa tgataaaaga aagttccgtg ggaaaggatt gcagaaaaga     180

ataaccgcga atacttttaa cgcctatatg agcagggcaa gaaagcggtt tgatgataaa     240

ttacatcata gctttgataa aaatattaat aaattatcgg aaaagtatcc tctttacagc     300

gaagaattat cttcatggct ttctatgcct acggctaata ttcgccagca catgtcatcg     360

ttacaatcta aattgaaaga aataatgccg cttgccgaag agttatcaaa tgtaagaata     420

ggctctaaag gcagtgatgc aaaaatagca agactaataa aaaaatatcc agattggagt     480

tttgctctta gtgatttaaa cagtgatgat tggaaggagc gccgtgacta tctttataag     540

ttattccaac aaggctctgc gttgttagaa gaactacacc agctcaaggt caaccatgag     600

gttctgtacc atctgcagct aagccctgcg gagcgtacat ctatacagca acgatgggcc     660

gatgttctgc gcgagaagaa gcgtaatgtt gtggttattg actacccaac atacatgcag     720

tctatctatg atattttgaa taatcctgcg actttattta gtttaaacac tcgttctgga     780

atggcacctt tggcctttgc tctggctgcg gtatcagggc gaagaatgat tgagataatg     840

tttcagggtg aatttgccgt ttcaggaaag tatacggtta atttctcagg gcaagctaaa     900

aaacgctctg aagataaaag cgtaaccaga acgatttata ctttatgcga agcaaaatta     960

ttcgttgaat tattaacaga attgcgttct tgctctgctg catctgattt cgatgaggtt    1020

gttaaaggat atggaaagga tgatacaagg tctgagaacg gcaggataaa tgctatttta    1080

gcaaaagcat ttaacccttg ggttaaatca tttttcggcg atgaccgtcg tgtttataaa    1140

gatagccgcg ctatttacgc tcgcatcgct tatgagatgt tcttccgcgt cgatccacgg    1200

tggaaaaacg tcgacgagga tgtgttcttc atggagattc tcggacacga cgatgagaac    1260

acccagctgc actataagca gttcaagctg gccaacttct ccagaacctg gcgacctgaa    1320

gttggggatg aaaacaccag gctggtggct ctgcagaaac tggacgatga aatgccaggc    1380

tttgccagag gtgacgctgg cgtccgtctc catgaaaccg ttaagcagct ggtggagcag    1440

gacccatcag caaaaataac caacagcact ctccgggcct ttaaatttag cccgacgatg    1500

attagccggt acctggagtt tgccgctgat gcattggggc agttcgttgg cgagaacggg    1560

cagtggcagc tgaagataga gacacctgca atcgtcctgc ctgatgaaga atccgttgag    1620

accatcgacg aaccggatga tgagtcccaa gacgacgagc tggatgaaga tgaaattgag 1680

ctcgacgagg gtggcggcga tgaaccaacc gaagaggaag ggccagaaga acatcagcca 1740

actgctctaa aacccgtctt caagcctgca aaaaataacg gggacggaac gtacaagata 1800

gagtttgaat acgatggaaa gcattatgcc tggtccggcc ccgccgatag ccctatggcc 1860

gcaatgcgat ccgcatggga aacgtactac agctaa 1896

<210> 12
<211> 631
<212> PRT
<213> Escherichia coli bacteriophage N15 protelomerase

<400> 12

Met Ser Lys Val Lys Ile Gly Glu Leu Ile Asn Thr Leu Val Asn Glu
1               5                   10                  15

Val Glu Ala Ile Asp Ala Ser Asp Arg Pro Gln Gly Asp Lys Thr Lys
            20                  25                  30

Arg Ile Lys Ala Ala Ala Ala Arg Tyr Lys Asn Ala Leu Phe Asn Asp
        35                  40                  45

Lys Arg Lys Phe Arg Gly Lys Gly Leu Gln Lys Arg Ile Thr Ala Asn
    50                  55                  60

Thr Phe Asn Ala Tyr Met Ser Arg Ala Arg Lys Arg Phe Asp Asp Lys
65                  70                  75                  80

Leu His His Ser Phe Asp Lys Asn Ile Asn Lys Leu Ser Glu Lys Tyr
                85                  90                  95

Pro Leu Tyr Ser Glu Glu Leu Ser Ser Trp Leu Ser Met Pro Thr Ala
            100                 105                 110

Asn Ile Arg Gln His Met Ser Ser Leu Gln Ser Lys Leu Lys Glu Ile
        115                 120                 125

Met Pro Leu Ala Glu Glu Leu Ser Asn Val Arg Ile Gly Ser Lys Gly
    130                 135                 140

Ser Asp Ala Lys Ile Ala Arg Leu Ile Lys Lys Tyr Pro Asp Trp Ser
145                 150                 155                 160

Phe Ala Leu Ser Asp Leu Asn Ser Asp Asp Trp Lys Glu Arg Arg Asp
                165                 170                 175

Tyr Leu Tyr Lys Leu Phe Gln Gln Gly Ser Ala Leu Leu Glu Glu Leu
            180                 185                 190

His Gln Leu Lys Val Asn His Glu Val Leu Tyr His Leu Gln Leu Ser
        195                 200                 205

Pro Ala Glu Arg Thr Ser Ile Gln Gln Arg Trp Ala Asp Val Leu Arg
    210                 215                 220

Glu Lys Lys Arg Asn Val Val Val Ile Asp Tyr Pro Thr Tyr Met Gln
225                 230                 235                 240

Ser Ile Tyr Asp Ile Leu Asn Asn Pro Ala Thr Leu Phe Ser Leu Asn
                245                 250                 255

Thr Arg Ser Gly Met Ala Pro Leu Ala Phe Ala Leu Ala Ala Val Ser
            260                 265                 270

Gly Arg Arg Met Ile Glu Ile Met Phe Gln Gly Glu Phe Ala Val Ser
        275                 280                 285

Gly Lys Tyr Thr Val Asn Phe Ser Gly Gln Ala Lys Lys Arg Ser Glu
    290                 295                 300

Asp Lys Ser Val Thr Arg Thr Ile Tyr Thr Leu Cys Glu Ala Lys Leu
305                 310                 315                 320

Phe Val Glu Leu Leu Thr Glu Leu Arg Ser Cys Ser Ala Ala Ser Asp
                325                 330                 335

Phe Asp Glu Val Val Lys Gly Tyr Gly Lys Asp Asp Thr Arg Ser Glu
            340                 345                 350

Asn Gly Arg Ile Asn Ala Ile Leu Ala Lys Ala Phe Asn Pro Trp Val
        355                 360                 365

Lys Ser Phe Phe Gly Asp Asp Arg Arg Val Tyr Lys Asp Ser Arg Ala
    370                 375                 380

Ile Tyr Ala Arg Ile Ala Tyr Glu Met Phe Phe Arg Val Asp Pro Arg
385                 390                 395                 400

Trp Lys Asn Val Asp Glu Asp Val Phe Phe Met Glu Ile Leu Gly His
                405                 410                 415

Asp Asp Glu Asn Thr Gln Leu His Tyr Lys Gln Phe Lys Leu Ala Asn
            420                 425                 430

Phe Ser Arg Thr Trp Arg Pro Glu Val Gly Asp Glu Asn Thr Arg Leu
        435                 440                 445

Val Ala Leu Gln Lys Leu Asp Asp Glu Met Pro Gly Phe Ala Arg Gly
    450                 455                 460

Asp Ala Gly Val Arg Leu His Glu Thr Val Lys Gln Leu Val Glu Gln
465                 470                 475                 480

Asp Pro Ser Ala Lys Ile Thr Asn Ser Thr Leu Arg Ala Phe Lys Phe
                485                 490                 495

Ser Pro Thr Met Ile Ser Arg Tyr Leu Glu Phe Ala Ala Asp Ala Leu
            500                 505                 510

Gly Gln Phe Val Gly Glu Asn Gly Gln Trp Gln Leu Lys Ile Glu Thr
        515                 520                 525

Pro Ala Ile Val Leu Pro Asp Glu Glu Ser Val Glu Thr Ile Asp Glu
    530                 535                 540

Pro Asp Asp Glu Ser Gln Asp Asp Glu Leu Asp Glu Asp Glu Ile Glu
545                 550                 555                 560

Leu Asp Glu Gly Gly Gly Asp Glu Pro Thr Glu Glu Glu Gly Pro Glu
                565                 570                 575

Glu His Gln Pro Thr Ala Leu Lys Pro Val Phe Lys Pro Ala Lys Asn
            580                 585                 590

Asn Gly Asp Gly Thr Tyr Lys Ile Glu Phe Glu Tyr Asp Gly Lys His
        595                 600                 605

Tyr Ala Trp Ser Gly Pro Ala Asp Ser Pro Met Ala Ala Met Arg Ser
    610                 615                 620

Ala Trp Glu Thr Tyr Tyr Ser
625                 630

<210>  13
<211>  1329
<212>  DNA
<213>  Agrobacterium tumefaciens str. C58 protelomerase

<400>  13
atgctcgccg caaaacgaaa aacaaaaaca ccggtcctcg tggaacgcat cgatcaattc      60

gtcggccaga tcaaagaggc gatgaaaagc gacgacgctt cgcgaaacag gaaaatccgc     120

gatctgtggg atgccgaggt ccgctatcat ttcgataatg gccgcacgga aaagacgctc     180

gaactttaca tcatgaaata tcgcaatgcg ctgaaggccg aattcggccc gaagagcacc     240

ccgctagcca tctgcaacat gaagaagctg cgcgagcgcc tgaacaccta tattgcccgg     300

ggcgattatc ccaagacagg cgtggcgacg tcgattgtcg aaaaaatcga gcgggcggag     360

ttcaacaccg ccggccgcaa acccacggtt ctccttcgca tagccgattt cattgccgcg 420

atgaacggca tggacgcgaa gcaggacatg caggctctgt gggacgcaga aatcgccatc 480

atgaacggcc gcgcccagac gacgatcatt tcctacatca ccaaatatcg caacgccatc 540

cgggaagcct tcggtgacga ccatccgatg ctgaagatcg ccaccggcga tgccgcgatg 600

tatgacgagg cccgccgggt gaagatggag aagatcgcca acaagcatgg cgcgctcatc 660

acattcgaga actaccggca ggttctgaaa atctgcgagg attgtctcaa gtccagcgat 720

cccctgatga tcggcatcgg cctcatcggc atgacgggac gccgacccta tgaagtcttc 780

acccaggcgg aattttcacc tgcaccctat ggcaagggag tttccaaatg gagcatcctg 840

ttcaacggtc aggccaagac gaaacagggc gagggcacga aattcgggat tacctatgaa 900

attcctgtcc tgacgcgctc cgaaaccgtg ctggctgcct ataagcgcct gcgcgaaagc 960

ggacagggaa aattatggca tggcatgtcg atcgacgatt tttcctcgga aacccgcctg 1020

ctgctgcgcg acacggtttt taacctgttc gaggatgtct ggccaaagga agagcttccc 1080

aagccctatg gcctcagaca cctctatgcc gaagtggcct atcacaattt cgcgccaccc 1140

catgtcacca agaacagcta tttcgccgcc attcttggcc acaacaataa cgacctcgaa 1200

acgtcgctgt cctatatgac ctatacgctg ccggaagacc gcgacaatgc gctggcgcgc 1260

ctgaagcgga ccaatgagcg gacattgcag caaatggcga cgatcgcgcc cgtgtcccgc 1320

aagggatga 1329

<210> 14
<211> 442
<212> PRT
<213> Agrobacterium tumefaciens str. C58 protelomerase

<400> 14

Met Leu Ala Ala Lys Arg Lys Thr Lys Thr Pro Val Leu Val Glu Arg
1               5                   10                  15

Ile Asp Gln Phe Val Gly Gln Ile Lys Glu Ala Met Lys Ser Asp Asp
            20                  25                  30

Ala Ser Arg Asn Arg Lys Ile Arg Asp Leu Trp Asp Ala Glu Val Arg
        35                  40                  45

Tyr His Phe Asp Asn Gly Arg Thr Glu Lys Thr Leu Glu Leu Tyr Ile
    50                  55                  60

Met Lys Tyr Arg Asn Ala Leu Lys Ala Glu Phe Gly Pro Lys Ser Thr
65                  70                  75                  80

Pro Leu Ala Ile Cys Asn Met Lys Lys Leu Arg Glu Arg Leu Asn Thr
                85                  90                  95

Tyr Ile Ala Arg Gly Asp Tyr Pro Lys Thr Gly Val Ala Thr Ser Ile
                100                 105                 110

Val Glu Lys Ile Glu Arg Ala Glu Phe Asn Thr Ala Gly Arg Lys Pro
        115                 120                 125

Thr Val Leu Leu Arg Ile Ala Asp Phe Ile Ala Ala Met Asn Gly Met
    130                 135                 140

Asp Ala Lys Gln Asp Met Gln Ala Leu Trp Asp Ala Glu Ile Ala Ile
145                 150                 155                 160

Met Asn Gly Arg Ala Gln Thr Thr Ile Ile Ser Tyr Ile Thr Lys Tyr
                165                 170                 175

Arg Asn Ala Ile Arg Glu Ala Phe Gly Asp Asp His Pro Met Leu Lys
            180                 185                 190

Ile Ala Thr Gly Asp Ala Ala Met Tyr Asp Glu Ala Arg Arg Val Lys
        195                 200                 205

Met Glu Lys Ile Ala Asn Lys His Gly Ala Leu Ile Thr Phe Glu Asn
    210                 215                 220

Tyr Arg Gln Val Leu Lys Ile Cys Glu Asp Cys Leu Lys Ser Ser Asp
225                 230                 235                 240

Pro Leu Met Ile Gly Ile Gly Leu Ile Gly Met Thr Gly Arg Arg Pro
                245                 250                 255

Tyr Glu Val Phe Thr Gln Ala Glu Phe Ser Pro Ala Pro Tyr Gly Lys
            260                 265                 270

Gly Val Ser Lys Trp Ser Ile Leu Phe Asn Gly Gln Ala Lys Thr Lys
        275                 280                 285

Gln Gly Glu Gly Thr Lys Phe Gly Ile Thr Tyr Glu Ile Pro Val Leu
    290                 295                 300

Thr Arg Ser Glu Thr Val Leu Ala Ala Tyr Lys Arg Leu Arg Glu Ser
305                 310                 315                 320

Gly Gln Gly Lys Leu Trp His Gly Met Ser Ile Asp Asp Phe Ser Ser
                325                 330                 335

Glu Thr Arg Leu Leu Leu Arg Asp Thr Val Phe Asn Leu Phe Glu Asp
            340                 345                 350

Val Trp Pro Lys Glu Glu Leu Pro Lys Pro Tyr Gly Leu Arg His Leu
        355                 360                 365

Tyr Ala Glu Val Ala Tyr His Asn Phe Ala Pro Pro His Val Thr Lys
    370                 375                 380

Asn Ser Tyr Phe Ala Ala Ile Leu Gly His Asn Asn Asn Asp Leu Glu
385                 390                 395                 400

Thr Ser Leu Ser Tyr Met Thr Tyr Thr Leu Pro Glu Asp Arg Asp Asn
                405                 410                 415

Ala Leu Ala Arg Leu Lys Arg Thr Asn Glu Arg Thr Leu Gln Gln Met
            420                 425                 430

Ala Thr Ile Ala Pro Val Ser Arg Lys Gly
        435                 440

<210>  15
<211>  26
<212>  DNA
<213>  Artificial Sequence

<220>
<223>  minimal substrate sequence for the Agrobacterium tumefaciens str.
       C58 protelomerase

<400>  15
aataacaata tcatgatatt gttatt                                          26

<210>  16
<211>  2631
<212>  DNA
<213>  DNA polymerase 1 of Geobacillus stearothermophilus

<400>  16
atgaagaaga agctagtact aattgatggc aacagtgtgg cataccgcgc cttttttgcc      60

ttgccacttt tgcataacga caaaggcatt catacgaatg cggtttacgg gtttacgatg     120

atgttgaaca aaattttggc ggaagaacaa ccgacccatt tacttgtagc gtttgacgcc     180

ggaaaaacga cgttccggca tgaaacgttt caagagtata aaggcggacg gcaacaaact     240

cccccggaac tgtccgagca gtttccgctg ttgcgcgagc tattaaaagc gtaccgcatt     300

cccgcttatg aacttgatca ttacgaagcg gacgatatta tcgggacgct cgctgcccgc     360

gctgagcaag aagggtttga agtgaaaatc atttccggcg accgcgattt aacccagctc     420

gcctcccgtc atgtgacggt cgatattacg aaaaaaggga ttaccgacat tgagccgtat     480

acgccagaga ccgttcgcga aaaatacggc ctgactccgg agcaaatagt ggatttaaaa     540

ggattgatgg gcgataaatc cgacaacatc ccgggcgtgc ccggcatcgg ggaaaaaacg     600

gcggtcaagc tgctgaagca atttggtacg gtggaaaatg tgctcgcatc gattgatgag     660

gtgaaagggg aaaaactgaa agaaaacttg cgccaacacc gggatttagc tctcttgagc 720

aaacagctgg cgtccatttg ccgcgacgcc ccggttgagc tgtcgttaga tgacattgtc 780

tacgaaggac aagaccgcga aaaagtcatc gcgttattta aagaactcgg gtttcagtcg 840

ttcttggaaa aaatggccgc gccggcagcc gaaggggaga aaccgcttga ggagatggag 900

tttgccatcg ttgacgtcat taccgaagag atgcttgccg acaaggcagc gcttgtcgtt 960

gaggtgatgg aagaaaacta ccacgatgcc ccgattgtcg gaatcgcact agtgaacgag 1020

catgggcgat tttttatgcg cccggagacc gcgctggctg attcgcaatt tttagcatgg 1080

cttgccgatg aaacgaagaa aaaaagcatg tttgacgcca agcgggcagt cgttgcctta 1140

aagtggaaag gaattgagct tcgcggcgtc gcctttgatt tattgctcgc tgcctatttg 1200

ctcaatccgg ctcaagatgc cggcgatatc gctgcggtgg cgaaaatgaa acaatatgaa 1260

gcggtgcggt cggatgaagc ggtctatggc aaaggcgtca agcggtcgct gccggacgaa 1320

cagacgcttg ctgagcatct cgttcgcaaa gcggcagcca tttgggcgct tgagcagccg 1380

tttatggacg atttgcggaa caacgaacaa gatcaattat taacgaagct tgagcagccg 1440

ctggcggcga ttttggctga aatggaattc actggggtga acgtggatac aaagcggctt 1500

gaacagatgg gttcggagct cgccgaacaa ctgcgtgcca tcgagcagcg catttacgag 1560

ctagccggcc aagagttcaa cattaactca ccaaaacagc tcggagtcat tttatttgaa 1620

aagctgcagc taccggtgct gaagaagacg aaaacaggct attcgacttc ggctgatgtg 1680

cttgagaagc ttgcgccgca tcatgaaatc gtcgaaaaca ttttgcatta ccgccagctt 1740

ggcaaactgc aatcaacgta tattgaagga ttgttgaaag ttgtgcgccc tgataccggc 1800

aaagtgcata cgatgttcaa ccaagcgctg acgcaaactg ggcggctcag ctcggccgag 1860

ccgaacttgc aaaacattcc gattcggctc gaagaggggc ggaaaatccg ccaagcgttc 1920

gtcccgtcag agccggactg gctcattttc gccgccgatt actcacaaat tgaattgcgc 1980

gtcctcgccc atatcgccga tgacgacaat ctaattgaag cgttccaacg cgatttggat 2040

attcacacaa aaacggcgat ggacattttc catgtgagcg aagaggaagt cacggccaac 2100

atgcgccgcc aggcaaaggc cgttaacttc ggtatcgttt acggaattag cgattacgga 2160

ttggcgcaaa acttgaacat tacgcgcaaa gaagctgccg aatttatcga acgttacttc 2220

gccagctttc cgggcgtaaa gcagtatatg gaaaacattg tgcaagaagc gaaacagaaa 2280

ggatatgtga caacgctgtt gcatcggcgc cgctatttgc ctgatattac aagccgcaat 2340

ttcaacgtcc gcagttttgc agagcggacg gccatgaaca cgccaattca aggaagcgcc 2400

gctgacatta ttaaaaaagc gatgattgat ttagcggcac ggctgaaaga agagcagctt 2460

caggctcgtc ttttgctgca agtgcatgac gagctcattt tggaagcgcc aaaagaggaa 2520

attgagcgat tatgtgagct tgttccggaa gtgatggagc aggccgttac gctccgcgtg 2580

ccgctgaaag tcgactacca ttacggccca acatggtatg atgccaaata a 2631

<210> 17
<211> 587
<212> PRT
<213> DNA Polymerase of Geobacillus stearothermophilus

<400> 17

Ala Glu Gly Glu Lys Pro Leu Glu Glu Met Glu Phe Ala Ile Val Asp
1               5                   10                  15

Val Ile Thr Glu Glu Met Leu Ala Asp Lys Ala Ala Leu Val Val Glu
            20                  25                  30

Val Met Glu Glu Asn Tyr His Asp Ala Pro Ile Val Gly Ile Ala Leu
        35                  40                  45

Val Asn Glu His Gly Arg Phe Phe Met Arg Pro Glu Thr Ala Leu Ala
    50                  55                  60

Asp Ser Gln Phe Leu Ala Trp Leu Ala Asp Glu Thr Lys Lys Lys Ser
65                  70                  75                  80

Met Phe Asp Ala Lys Arg Ala Val Val Ala Leu Lys Trp Lys Gly Ile
                85                  90                  95

Glu Leu Arg Gly Val Ala Phe Asp Leu Leu Leu Ala Ala Tyr Leu Leu
            100                 105                 110

Asn Pro Ala Gln Asp Ala Gly Asp Ile Ala Ala Val Ala Lys Met Lys
        115                 120                 125

Gln Tyr Glu Ala Val Arg Ser Asp Glu Ala Val Tyr Gly Lys Gly Val
    130                 135                 140

Lys Arg Ser Leu Pro Asp Glu Gln Thr Leu Ala Glu His Leu Val Arg
145                 150                 155                 160

Lys Ala Ala Ala Ile Trp Ala Leu Glu Gln Pro Phe Met Asp Asp Leu
                165                 170                 175

Arg Asn Asn Glu Gln Asp Gln Leu Leu Thr Lys Leu Glu Gln Pro Leu
            180                 185                 190

Ala Ala Ile Leu Ala Glu Met Glu Phe Thr Gly Val Asn Val Asp Thr
        195                 200                 205

Lys Arg Leu Glu Gln Met Gly Ser Glu Leu Ala Glu Gln Leu Arg Ala
    210                 215                 220

Ile Glu Gln Arg Ile Tyr Glu Leu Ala Gly Gln Glu Phe Asn Ile Asn
225                 230                 235                 240

Ser Pro Lys Gln Leu Gly Val Ile Leu Phe Glu Lys Leu Gln Leu Pro
                245                 250                 255

Val Leu Lys Lys Thr Lys Thr Gly Tyr Ser Thr Ser Ala Asp Val Leu
            260                 265                 270

Glu Lys Leu Ala Pro His His Glu Ile Val Glu Asn Ile Leu His Tyr
        275                 280                 285

Arg Gln Leu Gly Lys Leu Gln Ser Thr Tyr Ile Glu Gly Leu Leu Lys
    290                 295                 300

Val Val Arg Pro Asp Thr Gly Lys Val His Thr Met Phe Asn Gln Ala
305                 310                 315                 320

Leu Thr Gln Thr Gly Arg Leu Ser Ser Ala Glu Pro Asn Leu Gln Asn
                325                 330                 335

Ile Pro Ile Arg Leu Glu Glu Gly Arg Lys Ile Arg Gln Ala Phe Val
            340                 345                 350

Pro Ser Glu Pro Asp Trp Leu Ile Phe Ala Ala Asp Tyr Ser Gln Ile
        355                 360                 365

Glu Leu Arg Val Leu Ala His Ile Ala Asp Asp Asp Asn Leu Ile Glu
    370                 375                 380

Ala Phe Gln Arg Asp Leu Asp Ile His Thr Lys Thr Ala Met Asp Ile
385                 390                 395                 400

Phe His Val Ser Glu Glu Glu Val Thr Ala Asn Met Arg Arg Gln Ala
                405                 410                 415

Lys Ala Val Asn Phe Gly Ile Val Tyr Gly Ile Ser Asp Tyr Gly Leu
            420                 425                 430

Ala Gln Asn Leu Asn Ile Thr Arg Lys Glu Ala Ala Glu Phe Ile Glu
        435                 440                 445

Arg Tyr Phe Ala Ser Phe Pro Gly Val Lys Gln Tyr Met Glu Asn Ile
    450                 455                 460

Val Gln Glu Ala Lys Gln Lys Gly Tyr Val Thr Thr Leu Leu His Arg
465                 470                 475                 480

Arg Arg Tyr Leu Pro Asp Ile Thr Ser Arg Asn Phe Asn Val Arg Ser
                485                 490                 495

Phe Ala Glu Arg Thr Ala Met Asn Thr Pro Ile Gln Gly Ser Ala Ala
            500                 505                 510

Asp Ile Ile Lys Lys Ala Met Ile Asp Leu Ala Ala Arg Leu Lys Glu
        515                 520                 525

Glu Gln Leu Gln Ala Arg Leu Leu Leu Gln Val His Asp Glu Leu Ile
    530                 535                 540

Leu Glu Ala Pro Lys Glu Glu Ile Glu Arg Leu Cys Glu Leu Val Pro
545                 550                 555                 560

Glu Val Met Glu Gln Ala Val Thr Leu Arg Val Pro Leu Lys Val Asp
                565                 570                 575

Tyr His Tyr Gly Pro Thr Trp Tyr Asp Ala Lys
            580                 585

## Description

### FIELD OF THE INENTION

The invention is in the field of molecular biology, more in particular in the field of nucleic acid sequencing and amplification.

### BACKGROUND

Traditional cell-based processes for amplification of DNA in large quantities are costly. For example, use of bacteria requires their growth in large volumes in expensive fermenters that are required to be maintained in a sterile state in order to prevent contamination of the culture. The bacteria also need to be lysed to release the amplified DNA and the DNA needs to be cleaned and purified from other bacterial components, in particular, where amplification serves as a method for creating a template for detection and/or sequencing.

Further PCR amplification for example requires a thermal cycler and elevated incubation temperatures.

In the complex biochemical environment for example of the bacterial cell, it is difficult to control the quality and yields of the desired DNA product. The bacteria may occasionally alter the required gene cloned within the amplified DNA and render it useless for the required purpose. Recombination events may also lead to problems in faithful production of a DNA of interest.

Cell-free enzymatic processes for amplification of DNA avoid the requirement for use of a host cell, and so are advantageous.

The prior art (WO 2012/017210) discloses the use of at least a single species of primer for the amplification of a DNA template. The primer may be used for production of a linear covalently closed DNA (closed linear DNA). Here, the template DNA comprises at least one protelomerase target sequence. The primer binds specifically to a palindromic sequence within the at least one protelomerase target sequence and is allegedly capable of priming amplification in both directions. Only a single species of primer is required for the priming of each template. However this method has severe drawbacks if and when the amplification product is used for certain applications. One such drawback is that the template generated is not clonal, i.e. not a single nucleic acid chain is generated but multiple chains. This consequently means that solid phase applications may suffer from migrating and unbound nucleic acid molecules.

Paired-end sequencing is performed on linear DNA without concatemeric repeats. Alternatively, sequencing by ligation can be performed in both directions on a single-stranded DNA template. This has been used for sequencing by Complete Genomics (CG) (Radoje, D., Fredrik, D., Evan, H. & Fredrie, D. Nucleic Acid Sequencing and Process. (2011) and the Max-Seq on nanoballs/rolonies. An unusual pyrosequencing method based on RCA has been patented by Ion Torrent (Rothberg, J. et al. Methods for Sequencing Individual Nucleic Acids Under Tension. (2010) In order to optimise bidirectional sequencing by ligation, CG has developed a very complicated preparation method that regularly arranges short unknown inserts with constant regions in a circular template. Pacific Biosciences has disclosed single molecule real-time sequencing using the so-called SMRTbell as a template (Travers, K., Otto, G., Turner, S., Heiner, C. & Ma, C. COMPOSITIONS AND METHODS FOR NUCLEIC ACID SEQUENCING. (2009) at <http://www.freepatentsonline.com/y2009/0298075.html>). The SMRTbell is a circular product of a dsDNA that is read repetitively to provide a consensus sequence. This is not performed in two separate sequencing events as performed in paired-end sequencing. In so-called DNA "strand sequencing", Oxford Nanopore Technologies uses a double-stranded DNA construct with one terminal hairpin for single molecule sequencing by nanopores. By passing one strand through the nanopore, the sequence can be identified measuring the characteristic ionic current. The terminal hairpin allows the passage of the antisense strand once the sense strand is completely translocated, thus reducing the error rate by redundancy. So far, no method allows paired-end sequencing of longer inserts on rolling circle products.

WO 2012/017210 has further drawbacks. If both termini of a linear DNA fragment contain the protelomerase recognition sequence and are converted to hairpin ends by said protelomerase, these can not be sufficiently differentiated with a primer encompassing the recognition sequence alone. Thus, both ends can be amplified simultaneously resulting in at least two concatemeric strands generated from a single template. For sequencing it is preferred to have only one defined concatemeric product per template molecule using amplification primers that do not bind exclusively to the protelomerase recognition sequence. This allows a defined sequencing process that is initiated from primers that bind to known sequences preferably situated on opposite ends of the concatemer. Excess primer amounts also can result in the generation of branched non-covalently associated product formation. However correct sequencing can only occur on clonal concatemeric amplification products that are covalently linked and are not at risk to dissotiate during the sequencing process. Amplification primers can be modified in order to allow specific immobilisation of the resulting concatemeric product (nanoball). However, other immobilisation procedures such as crosslinking, hybridisation to oligonucleotides, or through non-covalent interactions such as hydrogen bonding, van der Waals forces, and the like (see US2011281736 lines 250-251)

Surprisingly an alternative method proves to work better and generates a single-stranded concatemeric nucleic acid chain, which is in itself self-complementary and may be bound to a solid phase. Such a molecule has numerous advantages over other nucleic acid templates.

### SUMMARY OF THE INVENTION

The present invention relates to a method for nucleic acid amplification, and detection or sequencing, comprising the steps of,
a. providing a double-stranded target nucleic acid molecule for amplification,
b. terminally attaching to said target nucleic acid molecule both up-stream and down-stream a first and second linker nucleic acid comprising a protelomerase target sequence,
c. reacting said target nucleic acid molecule with a protelomerase, preferably as encoded by SEQ ID NO.1, such that the target nucleic acid is converted to a circular target nucleic acid,
d. amplifying the circular target nucleic acid in an amplification reaction comprising a polymerase and at least one first primer which binds outside the protelomerase target sequence such that only one in parts self-complimentary amplification product is synthesized from one template.

The invention also relates to a kit for a method for nucleic acid amplification according to the invention, comprising a protelomerase enzyme as well as an amplification primer.

### SEQUENCES

| | |
|---|---|
| SEQ ID NO: 1 is the nucleic acid sequence of a Bacillus bacteriophage phi29 DNA polymerase. | ATGCCGAGAAAGATGTATAGTTGTGACTTTGAGACAACTACTAAAGTGGAA GACTGTAGGGTATGGGCGTATGGTTATATGAATATAGAAGATCACAGTGAG TACAAAATAGGTAATAGCCTGGATGAGTTTATGGCGTGGGTGTTGAAGGTA CAAGCTGATCTATATTTCCATAACCTCAAATTTGACGGAGCTTTTATCATTAA CTGGTTGGAACGTAATGGTTTTAAGTGGTCGGCTGACGGATTGCCAAACAC ATATAATACGATCATATCTCGCATGGGACAATGGTACATGATTGATATATGT TTAGGCTACAAAGGGAAACGTAAGATACATACAGTGATATATGACAGCTTA AAGAAACTACCGTTTCCTGTTAAGAAGATAGCTAAAGACTTTAAACTAACTG TTCTTAAAGGTGATATTGATTACCACAAAGAAAGACCAGTCGGCTATAAGAT AACACCCGAAGAATACGCCTATATTAAAAACGATATTCAGATTATTGCGGAA GCTCTGTTAATTCAGTTTAAGCAAGGTTTAGACCGGATGACAGCAGGCAGT GACAGTCTAAAAGGTTTCAAGGATATTATAACCACTAAGAAATTCAAAAAGG TGTTTCCTACATTGAGTCTTGGACTCGATAAGGAAGTGAGATACGCCTATAG AGGTGGTTTTACATGGTTAAATGATAGGTTCAAAGAAAAAGAAATCGGAGA AGGCATGGTCTTCGATGTTAATAGTCTATATCCTGCACAGATGTATAGCCGT CTCCTTCCATATGGTGAACCTATAGTATTCGAGGGTAAATACGTTTGGGACG AAGATTACCCACTACACATACAGCATATCAGATGTGAGTTCGAATTGAAAGA GGGCTATATACCCACTATACAGATAAAAAGAAGTAGGTTTTATAAAGGTAAT GAGTACCTAAAAAGTAGCGGCGGGGAGATAGCCGACCTCTGGTTGTCAAAT GTAGACCTAGAATTAATGAAAGAACACTACGATTTATATAACGTTGAATATA TCAGCGGCTTAAAATTTAAAGCAACTACAGGTTTGTTTAAAGATTTTATAGAT AAATGGACGTACATCAAGACGACATCAGAAGGAGCGATCAAGCAACTAGCA AAACTGATGTTAAACAGTCTATACGGTAAATTCGCTAGTAACCCTGATGTTA CAGGGAAAGTCCCTTATTTAAAAGAGAATGGGGCGCTAGGTTTCAGACTTG GAGAAGAGGAAACAAAAGACCCTGTTTATACACCTATGGGCGTTTTCATCAC TGCATGGGCTAGATACACGACAATTACAGCGGCACAGGCTTGTTATGATCG GATAATATACTGTGATACTGACAGCATACATTTAACGGGTACAGAGATACCT GATGTAATAAAAGATATAGTTGACCCTAAGAAATTGGGATACTGGGCACAT GAAAGTACATTCAAAAGAGCTAAATATCTGAGACAGAAGACCTATATACAA GACATCTATATGAAAGAAGTAGATGGTAAGTTAGTAGAAGGTAGTCCAGAT GATTACACTGATATAAAATTTAGTGTTAAATGTGCGGGAATGACTGACAAGA TTAAGAAAGAGGTTACGTTTGAGAATTTCAAAGTCGGATTCAGTCGGAAAA |
| | TGAAGCCTAAGCCTGTGCAAGTGCCGGGCGGGGTGGTTCTGGTTGATGACA CATTCACAATCAAATAA |
| SEQ ID NO: 2 is the amino acid sequence of a Bacillus bacteriophage phi29 DNA polymerase. | MPRKMYSCDFETTTKVEDCRVWAYGYMNIEDHSEYKIGNSLDEFMAWVLKV QADLYFHNLKFDGAFIINWLERNGFKWSADGLPNTYNTIISRMGQWYMIDICL GYKGKRKIHTVIYDSLKKLPFPVKKIAKDFKLTVLKGDIDYHKERPVGYKITPEEYA YIKNDIQIIAEALLIQFKQGLDRMTAGSDSLKGFKDIITTKKFKKVFPTLSLGLDKEV RYAYRGGFTWLNDRFKEKEIGEGMVFDVNSLYPAQMYSRLLPYGEPIVFEGKYV WDEDYPLHIQHIRCEFELKEGYIPTIQIKRSRFYKGNEYLKSSGGEIADLWLSNVD LELMKEHYDLYNVEYISGLKFKATTGLFKDFIDKWTYIKTTSEGAIKQLAKLMLNS LYGKFASNPDVTGKVPYLKENGALGFRLGEEETKDPVYTPMGVFITAWARYTTI TAAQACYDRIIYCDTDSIHLTGTEIPDVIKDIVDPKKLGYWAHESTFKRAKYLRQK TYIQDIYMKEVDGKLVEGSPDDYTDIKFSVKCAGMTDKIKKEVTFENFKVGFSRK MKPKPVQVPGGVVLVDDTFTIK |
| SEQ ID NO: 3 is the nucleic acid sequence of a Halomonas phage phiHAP-1 protelomerase nucleic acid sequence. | ATGAGCGGTGAGTCACGTAGAAAGGTCGATTTAGCGGAATTGATAGAGTG GTTGCTCAGCGAGATCAAAGATCGACGCCGATGATGAGATGCCACGTAA AGAGAAAACCAAGCGCATGGCGCGGCTGGCACGTAGCTTCAAAACGCGCCT GCATGATGACAAGCGCCGCAAGGATTCTGAGCGGATCGCGGTCACGACCTT TCGCCGCTACATGACAGAAGCGCGCAAGGCGGTGACTGCGCAGAACTGGC GCCATCACAGCTTCGACCAGCAGATCGAGCGGCTGGCCAGCCGCTACCCGG CTTATGCCAGCAAGCTGGAAGCGCTCGGCAAGCTGACCGATATCAGCGCCA TTCGTATGGCCCACCGCGAGCTGCTCGACCAGATCCGCAACGATGACGACG CTTATGAGGACATCCGGGCGATGAAGCTGGACCATGAAATCATGCGCCACC TGACGTTGAGCTCTGCACAGAAAAGCACGCTGGCTGAAGAGGCCAGCGAG ACGCTGGAAGAGCGCGCGGTGAACACGGTCGAGATCAACTACCACTGGTTG ATGGAGACGGTTTACGAGCTGCTGAGTAACCGGGAGAGAATGGTCGATGG GGAGTATCGCGGCTTTTTCAGTTACCTAGCGCTTGGGCTGGCGCTGGCCACC GGGCGTCGCTCGATCGAGGTGCTGAAGACCGGACGGATCACGAAGGTGGG CGAGTATGAGCTGGAGTTCAGCGGCCAGGCGAAAAAGCGCGGCGGCGTCG ACTATAGCGAGGCTTACCACATTTATACCCTGGTGAAAGCTGACCTGGTGAT CGAAGCGTGGGATGAGCTTCGCTCGCTGCCGGAAGCTGCTGAGCTGCAGG GCATGGACAACAGCGATGTGAACCGCCGCACGGCGAAGACGCTCAACACG CTCACTAAGCGGATCTTTAACAACGATGAGCGCGTTTTCAAGGACAGCCGG GCGATCTGGGCGCGGCTGGTGTTTGAGCTGCACTTCTCGCGCGACAAGCGC |
| | TGGAAGAAAGTCACCGAGGACGTGTTCTGGCGTGAGATGCTGGGGCATGA GGACATGGATACACAGCGCAGCTACCGCGCCTTTAAAATCGACTACGACGA GCCGGATCAAGCCGACCAGGAAGATTACGAACACGCTAGCCGCCTCGCCGC GCTGCAGGCGCTGGACGGCCATGAGCAGCTTGAGAGCAGCGACGCCCAGG CGCGTGTGCATGCCTGGGTGAAAGCGCAGATCGAGCAGGAGCCTGACGCG AAAATTACGCAGTCTCTGATCAGCCGGGAGCTGGGCGTTTATCGCCCTGCCA TAAAAGCGTACCTGGAGCTGGCGCGAGAGGCGCTCGACGCGCCGAACGTC GATCTGGACAAGGTCGCGGCGGCAGTGCCGAAGGAAGTAGCCGAGGCGAA GCCCCGGCTGAACGCCCACCCACAAGGGGATGGCAGGTGGGTCGGGGTGG CTTCAATCAACGGGGTGGAAGTTGCACGGGTGGGCAACCAGGCAGGCCGG ATCGAAGCGATGAAAGCGGCCTATAAAGCGGCGGGTGGGCGCTGA |
| SEQ ID NO: 4 is the amino acid sequence of a Halomonas phage phiHAP-1 15. | MSGESRRKVDLAELIEWLLSEIKEIDADDEMPRKEKTKRMARLARSFKTRLHDD KRRKDSERIAVTTFRRYMTEARKAVTAQNWRHHSFDQQIERLASRYPAYASKLE ALGKLTDISAIRMAHRELLDQIRNDDDAYEDIRAMKLDHEIMRHLTLSSAQKSTL AEEASETLEERAVNTVEINYHWLMETVYELLSNRERMVDGEYRGFFSYLALGLA LATGRRSIEVLKTGRITKVGEYELEFSGQAKKRGGVDYSEAYHIYTLVKADLVIEA WDELRSLPEAAELQGMDNSDVNRRTAKTLNTLTKRIFNNDERVFKDSRAIWAR LVFELHFSRDKRWKKVTEDVFWREMLGHEDMDTQRSYRAFKIDYDEPDQADQ EDYEHASRLAALQALDGHEQLESSDAQARVHAWVKAQIEQEPDAKITQSLISRE LGVYRPAIKAYLELAREALDAPNVDLDKVAAAVPKEVAEAKPRLNAHPQGDGR WVGVASINGVEVARVGNQAGRIEAMKAAYKAAGGR |
| SEQ ID NO: 5 is the nucleic acid sequence of a Yersinia phage PY54 protelomerase. | ATGGGTTACTTATTATTTTTATTTGGAGGCGTTATGAAAATCCATTTTCGCGA TTTAGTTAGTGGTTTAGTTAAAGAGATCGATGAAATAGAAAAATCAGACCG GGCGCAGGGTGACAAAACTCGGCGTTATCAGGGCGCGGCCAGAAAGTTCA AAAATGCCGTGTTTATGGATAAACGGAAATATCGCGGTAACGGTATGAAGA ATAGAATATCGTTAACAACATTTAATAAATATTTAAGTCGAGCACGTTCTCG GTTTGAAGAAAGGCTTCACCATAGTTTTCCTCAATCTATAGCAACTATCTCAA ATAAATATCCTGCATTCAGCGAAATAATAAAAGATCTGGATAATAGACCCGC TCATGAAGTTAGAATAAAACTTAAAGAATTAATAACTCATCTTGAATCCGGT GTTAATTTATTAGAAAAAATAGGTAGCTTAGGGAAAATAAAACCATCTACAG CTAAAAAAATAGTTAGCTTAAAAAAAATGTACCCATCATGGGCTAATGATCT AGATACTTTAATTAGTACTGAAGATGCTACAGAATTACAACAAAAGTTAGAG CAAGGGACCGACCTACTTAACGCATTACATTCTCTAAAAGTAAACCATGAAG |
| | TTATGTATGCATTAACGATGCAGCCTTCTGACAGAGCTGCATTAAAAGCTAG GCATGACGCTGCCCTTCACTTTAAAAAGCGTAACATCGTACCTATCGATTATC CCGGCTATATGCAACGAATGACGGACATACTACATCTTCCAGATATAGCTTT TGAAGATTCGATGGCATCACTTGCCCCTTTAGCATTTGCTCTAGCAGCTGCTA GCGGTCGCAGACAAATTGAAATACTAATTACTGGTGAGTTTGACGCCAAAA ATAAAAGCATCATTAAATTTTCTGGACAAGCAAAAAAAAGAATGGCCGTTTC AGGTGGACATTATGAAATATACAGTCTAATTGACTCAGAGCTATTCATTCAA CGGTTAGAGTTTTTACGTTCTCATAGCTCAATACTTCGATTACAAAATTTGGA AATAGCACATGATGAACATCGTACTGAACTATCTGTTATTAACGGTTTTGTA GCCAAACCTTTAAATGATGCAGCAAAACAGTTCTTTGTCGATGACAGAAGAG TATTTAAAGATACCCGTGCAATTTACGCTCGCATAGCATATGAAAAATGGTT TAGAACAGATCCTCGCTGGGCGAAGTGCGACGAAGATGTTTTCTTCTCTGAA TTATTAGGCCATGACGACCCAGATACTCAGCTGGCATATAAACAATTCAAGC TGGTAAATTTCAATCCAAAATGGACACCTAATATATCAGATGAAAACCCTCG GTTAGCTGCACTTCAAGAGCTTGACAATGATATGCCCGGCCTAGCACGTGGC GATGCGGCAGTTCGCATACATGAGTGGGTTAAAGAGCAACTGGCGCAGAAC CCTGCGGCAAAAATAACTGCATACCAAATCAAGAAAAATTTAAATTGTCGAA ATGACTTGGCCAGCCGATACATGGCATGGTGTGCTGACGCGCTAGGGGTTG TTATTGGTGATGATGGACAGGCAAGGCCAGAAGAACTCCCACCATCGCTCG TGCTTGATATTAACGCTGATGACACTGACGCTGAAGAAGATGAAATAGAGG AAGACTTTACTGATGAGGAAATAGACGACACCGAATTCGACGTATCAGATA ACGCCAGTGATGAAGATAAGCCCGAAGATAAACCTCGCTTTGCAGCACCAA TTCGTAGAAGTGAGGACTCTTGGCTGATTAAATTTGAATTTGCTGGCAAGCA ATATAGCTGGGAGGGTAATGCCGAAAGTGTTATCGATGCGATGAAACAAGC ATGGACTGAAAATATGGAGTAA |
| SEQ ID NO: 6 is the amino acid sequence of a Yersinia phage PY54. | MGYLLFLFGGVMKIHFRDLVSGLVKEIDEIEKSDRAQGDKTRRYQGAARKFKNA VFMDKRKYRGNGMKNRISLTTFNKYLSRARSRFEERLHHSFPQSIATISNKYPAF SEIIKDLDNRPAHEVRIKLKELITHLESGVNLLEKIGSLGKIKPSTAKKIVSLKKMYPS WANDLDTLISTEDATELQQKLEQGTDLLNALHSLKVNHEVMYALTMQPSDRA ALKARHDAALHFKKRNIVPIDYPGYMQRMTDILHLPDIAFEDSMASLAPLAFAL AAASGRRQIEILITGEFDAKNKSIIKFSGQAKKRMAVSGGHYEIYSLIDSELFIQRLE FLRSHSSILRLQNLEIAHDEHRTELSVINGFVAKPLNDAAKQFFVDDRRVFKDTR AIYARIAYEKWFRTDPRWAKCDEDVFFSELLGHDDPDTQLAYKQFKLVNFNPK WTPNISDENPRLAALQELDNDMPGLARGDAAVRIHEWVKEQLAQNPAAKITA |
| | YQIKKNLNCRNDLASRYMAWCADALGVVIGDDGQARPEELPPSLVLDINADDT DAEEDEIEEDFTDEEIDDTEFDVSDNASDEDKPEDKPRFAAPIRRSEDSWLIKFEF AGKQYSWEGNAESVIDAMKQAWTENME |
| SEQ ID NO: 7 is the nucleic acid sequence of a Klebsiella phage phiK02 protelomerase. | ATGCGTAAGGTGAAAATTGGTGAGCTAATCAATTCGCTTGTGAGCGAGGTC GAGGCAATCGATGCCTCTGATCGTCCGCAAGGCGATAAAACGAAGAAAATT AAAGCCGCAGCATTAAAATATAAGAATGCATTATTTAATGACAAAAGAAAGT TTCGCGGTAAAGGTTTAGAAAAAAGAATTTCTGCCAACACGTTCAACTCGTA TATGAGTCGGGCAAGGAAAAGATTTGATGATAGATTGCATCATAACTTTGA AAAGAATGTAATTAAACTATCAGAAAAATATCCTTTATATAGTGAAGAATTA TCTTCGTGGCTTTCTATGCCTGCGGCATCAATTAGACAGCATATGTCAAGATT GCAAGCCAAGCTAAAAGAGATAATGCCATTGGCAGAAGACTTATCCAATAT AAAGATTGGTACAAAAAATAGCGAAGCAAAAATAAATAAACTCGCTAATAA ATATCCTGAATGGCAATTCGCTATTAGTGATTTAAATAGCGAAGATTGGAAG GATAAAAGAGATTATCTTTATAAACTATTCCAACAAGGTTCTTCGCTCCTGGA AGACTTGAATAACCTGAAAGTAAACCATGAGGTTCTCTATCATCTGCAGCTT AGTTCTGCCGAGCGAACCTCTATCCAGCAGCGCTGGGCCAACGTCCTCAGCG AGAAAAAGCGCAACGTTGTCGTGATTGACTATCCGCGCTATATGCAGGCCAT CTACGATATAATCAACAAGCCTATAGTTTCGTTCGATTTGACTACTCGTCGTG GTATGGCCCCGCTGGCGTTCGCCCTTGCCGCGCTATCTGGTCGCCGAATGAT TGAAATCATGCTCCAGGGTGAATTTTCCGTCGCAGGTAAATATACAGTAACA TTCCTGGGGCAAGCTAAAAAACGCTCGGAAGATAAAGGTATATCAAGGAAA ATATATACCTTATGCGACGCTACTTTATTTGTTAGTTTGGTAAATGAACTTCG CTCATGCCCCGCTGCTGCGGATTTTGATGAAGTAATAAAAGGATATGGCGA AAATGACACTCGCTCAGAAAATGGGCGTATTAATGCAATTCTCGCTACAGCT TTTAATCCGTGGGTAAAAACTTTCTTAGGCGATGACCGCCGCGTTTATAAAG ATAGCCGCGCTATTTACGCCCGTATTGCCTATGAAATGTTCTTCCGCGTTGAC CCTCGGTGGAAGAATGTTGATGAGGATGTATTCTTCATGGAGATTCTCGGCC ATGACGATGAAAACACCCAACTGCACTATAAGCAGTTTAAATTGGCTAACTT CTCCAGAACATGGCGACCAAATGTCGGCGAGGAGAATGCCCGCCTAGCGGC GCTGCAAAAGCTGGATAGCATGATGCCAGATTTTGCCAGGGGCGACGCCGG GGTTCGTATTCATGAGACCGTGAAGCAGCTGGTGGAGCAGGACCCATCGAT AAAAATCACAAACAGCACCCTGCGACCGTTTAACTTCAGTACCAGGCTGATT CCTCGCTACCTGGAGTTTGCCGCCGATGCATTGGGCCAGTTCGTCGGTGAAA ATGGGCAATGGCAACTGAAGGATGAGGCGCCTGCAATAGTCCTGCCTGATG |
| | AGGAAATTCTTGAGCCTATGGACGACGTCGATCTCGATGACGAAAACCATG ATGATGAAACGCTGGATGACGATGAGATCGAAGTGGACGAAAGCGAAGGA GAGGAACTGGAGGAAGCGGGCGACGCTGAAGAGGCCGAGGTGGCTGAAC AGGAAGAGAAGCACCCTGGCAAGCCAAACTTTAAAGCGCCGAGGGATAAT GGCGATGGTACCTACATGGTGGAATTTGAATTCGGTGGCCGTCATTACGCCT GGTCCGGTGCCGCCGGTAATCGGGTAGAGGCAATGCAATCTGCCTGGAGTG CCTACTTCAAGTGA |
| SEQ ID NO: 8 is the amino acid sequence of a Klebsiella phage phiK02 protelomerase. | MRKVKIGELINSLVSEVEAIDASDRPQGDKTKKIKAAALKYKNALFNDKRKFRGK GLEKRISANTFNSYMSRARKRFDDRLHHNFEKNVIKLSEKYPLYSEELSSWLSMP AASIRQHMSRLQAKLKEIMPLAEDLSNIKIGTKNSEAKINKLANKYPEWQFAISD LNSEDWKDKRDYLYKLFQQGSSLLEDLNNLKVNHEVLYHLQLSSAERTSIQQRW ANVLSEKKRNVVVIDYPRYMQAIYDIINKPIVSFDLTTRRGMAPLAFALAALSGR RMIEIMLQGEFSVAGKYTVTFLGQAKKRSEDKGISRKIYTLCDATLFVSLVNELRS CPAAADFDEVIKGYGENDTRSENGRINAILATAFNPWVKTFLGDDRRVYKDSRA IYARIAYEMFFRVDPRWKNVDEDVFFMEILGHDDENTQLHYKQFKLANFSRTW RPNVGEENARLAALQKLDSMMPDFARGDAGVRIHETVKQLVEQDPSIKITNST LRPFNFSTRLIPRYLEFAADALGQFVGENGQWQLKDEAPAIVLPDEEILEPMDD VDLDDENHDDETLDDDEIEVDESEGEELEEAGDAEEAEVAEQEEKHPGKPNFK APRDNGDGTYMVEFEFGGRHYAWSGAAGNRVEAMQSAWSAYFK |
| SEQ ID NO: 9 is the nucleic acid sequence of a Vibrio phage VP882 protelomerase. | ATGAGCGGCGAAAGTAGACAAAAGGTAAACCTCGAGGAGTTAATAAATGA GCTCGTCGAGGAGGTGAAAACCATCGATGACAATGAGGCGATTACTCGGTC TGAAAAAACCAAGTTGATCACCAGGGCGGCGACTAAATTCAAGACCAAGCT GCACGACGATAAGCGCCGGAAGGATGCGACCAGAATCGCTCTGAGCACCTA TCGTAAGTACATGACAATGGCCAGGGCAGCAGTTACTGAGCAGAACTGGAA ACACCACAGTCTCGAGCAGCAGATAGAGCGGCTGGCCAAAAAGCACCCGCA ATACGCTGAGCAGCTGGTGGCCATCGGGGCCATGGATAACATCACCGAGTT GCGCCTGGCGCATCGCGACCTCCTGAAGAGCATCAAGGACAACGATGAAGC CTTCGAGGATATCCGCAGCATGAAGTTAGACCACGAGGTAATGCGCCATCT GACGCTACCCAGTGCGCAAAAGGCGAGACTGGCAGAGGAAGCCGCCGAGG CGTTGACCGAGAAGAAAACCGCCACGGTCGACATCAACTATCACGAGCTGA TGGCCGGCGTGGTGGAGCTGTTGACCAAGAAGACCAAGACGGTCGGCAGC GACAGCACCTACAGCTTCAGCCGGCTGGCGCTTGGTATTGGCCTGGCTACC GGTCGTCGTTCTATCGAGATACTGAAGCAGGGCGAGTTCAAAAAGGTGGAT |
| | GAGCAGCGGCTCGAGTTCTCTGGCCAAGCGAAAAAGCGCGGCGGTGCCGA CTATTCAGAGACCTATACCATTTACACCCTGGTCGACTCCGACCTGGTACTGA TGGCGCTGAAGAACCTGCGAGAGTTGCCAGAAGTTCGCGCACTGGATGAGT ACGACCAACTGGGCGAGATTAAGCGGAACGACGCCATCAATAAACGCTGTG CAAAAACGCTCAACCAAACCGCCAAGCAGTTCTTTGGCAGCGACGAGCGCG TGTTCAAAGATAGTCGTGCCATCTGGGCGCGTCTGGCTTATGAGTTGTTTTT CAACGTGATCCGCGCTGGAAAAAGAAAGACGAGGACGTTTTCTGGCAGGA GATGCTGGGCCACGAGGACATCGAGACTCAGAAAGCCTATAAGCAATTCAA GGTCGACTACAGCGAACCTGAGCAGCCGGTGCACAAGCCTGGCAAATTTAA GAGCAGAGCTGAAGCCCTCGCGGCGCTCGACTCAAATGAGGACATTACCAC CCGCTCATCCATGGCCAAGATCCACGACTGGGTGAAAGAGCGTATTGCGGA AGACCCCGAGGCGAACATCACACAGTCACTCATCACCCGGGAACTGGGCTC AGGCCGTAAGGTGATCAAGGACTACCTCGACCTGGCTGACGATGCCCTTGC TGTGGTGAATACTCCTGTCGATGACGCAGTCGTCGAGGTTCCAGCTGATGTG CCGGCAGCAGAAAAACAGCCGAAGAAAGCGCAGAAGCCCAGACTCGTGGC TCACCAGGTTGATGATGAGCACTGGGAAGCCTGGGCGCTGGTGGAAGGCG AGGAGGTGGCCAGGGTGAAAATCAAGGGCACCCGCGTTGAGGCAATGACA GCCGCATGGGAGGCCAGCCAAAAGGCACTCGATGACTAA |
| SEQ ID NO: 10 is the amino acid sequence of a Vibrio phage VP882 protelomerase. | MSGESRQKVNLEELINELVEEVKTIDDNEAITRSEKTKLITRAATKFKTKLHDDKR RKDATRIALSTYRKYMTMARAAVTEQNWKHHSLEQQIERLAKKHPQYAEQLV AIGAMDNITELRLAHRDLLKSIKDNDEAFEDIRSMKLDHEVMRHLTLPSAQKAR LAEEAAEALTEKKTATVDINYHELMAGVVELLTKKTKTVGSDSTYSFSRLALGIGL ATGRRSIEILKQGEFKKVDEQRLEFSGQAKKRGGADYSETYTIYTLVDSDLVLMA LKNLRELPEVRALDEYDQLGEIKRNDAINKRCAKTLNQTAKQFFGSDERVFKDSR AIWARLAYELFFQRDPRWKKKDEDVFWQEMLGHEDIETQKAYKQFKVDYSEP EQPVHKPGKFKSRAEALAALDSNEDITTRSSMAKIHDWVKERIAEDPEANITQSL ITRELGSGRKVIKDYLDLADDALAVVNTPVDDAVVEVPADVPAAEKQPKKAQKP RLVAHQVDDEHWEAWALVEGEEVARVKIKGTRVEAMTAAWEASQKALDD |
| SEQ ID NO: 11 is the nucleic acid sequence of an Escherichia coli bacteriophage N15 protelomerase. | ATGAGCAAGGTAAAAATCGGTGAGTTGATCAACACGCTTGTGAATGAGGTA GAGGCAATTGATGCCTCAGACCGCCCACAAGGCGACAAAACGAAGAGAATT AAAGCCGCAGCCGCACGGTATAAGAACGCGTTATTTAATGATAAAAGAAAG TTCCGTGGGAAAGGATTGCAGAAAAGAATAACCGCGAATACTTTTAACGCCT ATATGAGCAGGGCAAGAAAGCGGTTTGATGATAAATTACATCATAGCTTTGA |
| | TAAAAATATTAATAAATTATCGGAAAAGTATCCTCTTTACAGCGAAGAATTAT CTTCATGGCTTTCTATGCCTACGGCTAATATTCGCCAGCACATGTCATCGTTA CAATCTAAATTGAAAGAAATAATGCCGCTTGCCGAAGAGTTATCAAATGTAA GAATAGGCTCTAAAGGCAGTGATGCAAAAATAGCAAGACTAATAAAAAAAT ATCCAGATTGGAGTTTTGCTCTTAGTGATTTAAACAGTGATGATTGGAAGGA GCGCCGTGACTATCTTTATAAGTTATTCCAACAAGGCTCTGCGTTGTTAGAAG AACTACACCAGCTCAAGGTCAACCATGAGGTTCTGTACCATCTGCAGCTAAG CCCTGCGGAGCGTACATCTATACAGCAACGATGGGCCGATGTTCTGCGCGA GAAGAAGCGTAATGTTGTGGTTATTGACTACCCAACATACATGCAGTCTATC TATGATATTTTGAATAATCCTGCGACTTTATTTAGTTTAAACACTCGTTCTGGA ATGGCACCTTTGGCCTTTGCTCTGGCTGCGGTATCAGGGCGAAGAATGATTG AGATAATGTTTCAGGGTGAATTTGCCGTTTCAGGAAAGTATACGGTTAATTT CTCAGGGCAAGCTAAAAAACGCTCTGAAGATAAAAGCGTAACCAGAACGAT TTATACTTTATGCGAAGCAAAATTATTCGTTGAATTATTAACAGAATTGCGTT CTTGCTCTGCTGCATCTGATTTCGATGAGGTTGTTAAAGGATATGGAAAGGA TGATACAAGGTCTGAGAACGGCAGGATAAATGCTATTTTAGCAAAAGCATTT AACCCTTGGGTTAAATCATTTTTCGGCGATGACCGTCGTGTTTATAAAGATAG CCGCGCTATTTACGCTCGCATCGCTTATGAGATGTTCTTCCGCGTCGATCCAC GGTGGAAAAACGTCGACGAGGATGTGTTCTTCATGGAGATTCTCGGACACG ACGATGAGAACACCCAGCTGCACTATAAGCAGTTCAAGCTGGCCAACTTCTC CAGAACCTGGCGACCTGAAGTTGGGGATGAAAACACCAGGCTGGTGGCTCT GCAGAAACTGGACGATGAAATGCCAGGCTTTGCCAGAGGTGACGCTGGCGT CCGTCTCCATGAAACCGTTAAGCAGCTGGTGGAGCAGGACCCATCAGCAAA AATAACCAACAGCACTCTCCGGGCCTTTAAATTTAGCCCGACGATGATTAGC CGGTACCTGGAGTTTGCCGCTGATGCATTGGGGCAGTTCGTTGGCGAGAAC GGGCAGTGGCAGCTGAAGATAGAGACACCTGCAATCGTCCTGCCTGATGAA GAATCCGTTGAGACCATCGACGAACCGGATGATGAGTCCCAAGACGACGAG CTGGATGAAGATGAAATTGAGCTCGACGAGGGTGGCGGCGATGAACCAACC GAAGAGGAAGGGCCAGAAGAACATCAGCCAACTGCTCTAAAACCCGTCTTC AAGCCTGCAAAAAATAACGGGGACGGAACGTACAAGATAGAGTTTGAATAC GATGGAAAGCATTATGCCTGGTCCGGCCCCGCCGATAGCCCTATGGCCGCAA TGCGATCCGCATGGGAAACGTACTACAGCTAA |
| SEQ ID NO: 12 is the amino acid sequence of | MSKVKIGELINTLVNEVEAIDASDRPQGDKTKRIKAAAARYKNALFNDKRKFRGK GLQKRITANTFNAYMSRARKRFDDKLHHSFDKNINKLSEKYPLYSEELSSWLSMP |
| an Escherichia coli bacteriophage N15 protelomerase. | TANIRQHMSSLQSKLKEIMPLAEELSNVRIGSKGSDAKIARLIKKYPDWSFALSDL NSDDWKERRDYLYKLFQQGSALLEELHQLKVNHEVLYHLQLSPAERTSIQQRWA DVLREKKRNVVVIDYPTYMQSIYDILNNPATLFSLNTRSGMAPLAFALAAVSGRR MIEIMFQGEFAVSGKYTVNFSGQAKKRSEDKSVTRTIYTLCEAKLFVELLTELRSC SAASDFDEVVKGYGKDDTRSENGRINAILAKAFNPWVKSFFGDDRRVYKDSRAI YARIAYEMFFRVDPRWKNVDEDVFFMEILGHDDENTQLHYKQFKLANFSRTW RPEVGDENTRLVALQKLDDEMPGFARGDAGVRLHETVKQLVEQDPSAKITNST LRAFKFSPTMISRYLEFAADALGQFVGENGQWQLKIETPAIVLPDEESVETIDEPD DESQDDELDEDEIELDEGGGDEPTEEEGPEEHQPTALKPVFKPAKNNGDGTYKI EFEYDGKHYAWSGPADSPMAAMRSAWETYYS |
| SEQ ID NO: 13 is the nucleic acid sequence of the Agrobacterium tumefaciens str. C58 protelomerase. | ATGCTCGCCGCAAAACGAAAAACAAAAACACCGGTCCTCGTGGAACGCATC GATCAATTCGTCGGCCAGATCAAAGAGGCGATGAAAAGCGACGACGCTTCG CGAAACAGGAAAATCCGCGATCTGTGGGATGCCGAGGTCCGCTATCATTTCG ATAATGGCCGCACGGAAAGACGCTCGAACTTTACATCATGAAATATCGCAA TGCGCTGAAGGCCGAATTCGGCCCGAAGAGCACCCCGCTAGCCATCTGCAA CATGAAGAAGCTGCGCGAGCGCCTGAACACCTATATTGCCCGGGGCGATTA TCCCAAGACAGGCGTGGCGACGTCGATTGTCGAAAAAATCGAGCGGGCGGA GTTCAACACCGCCGGCCGCAAACCCACGGTTCTCCTTCGCATAGCCGATTTCA TTGCCGCGATGAACGGCATGGACGCGAAGCAGGACATGCAGGCTCTGTGGG ACGCAGAAATCGCCATCATGAACGGCCGCGCCCAGACGACGATCATTTCCTA CATCACCAAATATCGCAACGCCATCCGGGAAGCCTTCGGTGACGACCATCCG ATGCTGAAGATCGCCACCGGCGATGCCGCGATGTATGACGAGGCCCGCCGG GTGAAGATGGAGAAGATCGCCAACAAGCATGGCGCGCTCATCACATTCGAG AACTACCGGCAGGTTCTGAAAATCTGCGAGGATTGTCTCAAGTCCAGCGATC CCCTGATGATCGGCATCGGCCTCATCGGCATGACGGACGCCGACCCTATGA AGTCTTCACCCAGGCGGAATTTTCACCTGCACCCTATGGCAAGGGAGTTTCC AAATGGAGCATCCTGTTCAACGGTCAGGCCAAGACGAAACAGGGCGAGGG CACGAAATTCGGGATTACCTATGAAATTCCTGTCCTGACGCGCTCCGAAACC GTGCTGGCTGCCTATAAGCGCCTGCGCGAAAGCGGACAGGGAAAATTATGG CATGGCATGTCGATCGACGATTTTTCCTCGGAAACCCGCCTGCTGCTGCGCG ACACGGTTTTTAACCTGTTCGAGGATGTCTGGCCAAAGGAAGAGCTTCCCAA GCCCTATGGCCTCAGACACCTCTATGCCGAAGTGGCCTATCACAATTTCGCGC CACCCCATGTCACCAAGAACAGCTATTTCGCCGCCATTCTTGGCCACAACAAT AACGACCTCGAAACGTCGCTGTCCTATATGACCTATACGCTGCCGGAAGACC |
| | GCGACAATGCGCTGGCGCGCCTGAAGCGGACCAATGAGCGGACATTGCAGC AAATGGCGACGATCGCGCCCGTGTCCCGCAAGGGATGA |
| SEQ ID NO: 14 is the amino acid sequence of the Agrobacterium tumefaciens str. C58 protelomerase. | MLAAKRKTKTPVLVERIDQFVGQIKEAMKSDDASRNRKIRDLWDAEVRYHFDN GRTEKTLELYIMKYRNALKAEFGPKSTPLAICNMKKLRERLNTYIARGDYPKTGV ATSIVEKIERAEFNTAGRKPTVLLRIADFIAAMNGMDAKQDMQALWDAEIAIM NGRAQTTIISYITKYRNAIREAFGDDHPMLKIATGDAAMYDEARRVKMEKIANK HGALITFENYRQVLKICEDCLKSSDPLMIGIGLIGMTGRRPYEVFTQAEFSPAPYG KGVSKWSILFNGQAKTKQGEGTKFGITYEIPVLTRSETVLAAYKRLRESGQGKLW HGMSIDDFSSETRLLLRDTVFNLFEDVWPKEELPKPYGLRHLYAEVAYHNFAPP HVTKNSYFAAILGHNNNDLETSLSYMTYTLPEDRDNALARLKRTNERTLQQMA TIAPVSRKG |
| SEQ ID NO: 15 is the minimal substrate sequence for the Agrobacterium tumefaciens str. C58 protelomerase. | AATAACAATATCATGATATTGTTATT |
| SEQ ID NO: 16 is the nucleic acid sequence of the Bst DNA Polymerase (DNA Polymerase I of Geobacillus stearothermophilus) | ATGAAGAAGAAGCTAGTACTAATTGATGGCAACAGTGTGGCATACCGCGCC TTTTTTGCCTTGCCACTTTTGCATAACGACAAAGGCATTCATACGAATGCGGT TTACGGGTTTACGATGATGTTGAACAAAATTTTGGCGGAAGAACAACCGAC CCATTTACTTGTAGCGTTTGACGCCGGAAAAACGACGTTCCGGCATGAAACG TTTCAAGAGTATAAAGGCGGACGGCAACAAACTCCCCCGGAACTGTCCGAG CAGTTTCCGCTGTTGCGCGAGCTATTAAAAGCGTACCGCATTCCCGCTTATG AACTTGATCATTACGAAGCGGACGATATTATCGGGACGCTCGCTGCCCGCGC TGAGCAAGAAGGGTTTGAAGTGAAAATCATTTCCGGCGACCGCGATTTAAC CCAGCTCGCCTCCCGTCATGTGACGGTCGATATTACGAAAAAAGGGATTACC GACATTGAGCCGTATACGCCAGAGACCGTTCGCGAAAAATACGGCCTGACT CCGGAGCAAATAGTGGATTTAAAAGGATTGATGGGCGATAAATCCGACAAC ATCCCGGGCGTGCCCGGCATCGGGGAAAAAACGGCGGTCAAGCTGCTGAA GCAATTTGGTACGGTGGAAAATGTGCTCGCATCGATTGATGAGGTGAAAGG GGAAAAACTGAAAGAAAACTTGCGCCAACACCGGGATTTAGCTCTCTTGAG CAAACAGCTGGCGTCCATTTGCCGCGACGCCCCGGTTGAGCTGTCGTTAGAT GACATTGTCTACGAAGGACAAGACCGCGAAAAAGTCATCGCGTTATTTAAA |
| | GAACTCGGGTTTCAGTCGTTCTTGGAAAAAATGGCCGCGCCGGCAGCCGAA GGGGAGAAACCGCTTGAGGAGATGGAGTTTGCCATCGTTGACGTCATTACC GAAGAGATGCTTGCCGACAAGGCAGCGCTTGTCGTTGAGGTGATGGAAGA AAACTACCACGATGCCCCGATTGTCGGAATCGCACTAGTGAACGAGCATGG GCGATTTTTTATGCGCCCGGAGACCGCGCTGGCTGATTCGCAATTTTTAGCA TGGCTTGCCGATGAAACGAAGAAAAAAAGCATGTTTGACGCCAAGCGGGCA GTCGTTGCCTTAAAGTGGAAAGGAATTGAGCTTCGCGGCGTCGCCTTTGATT TATTGCTCGCTGCCTATTTGCTCAATCCGGCTCAAGATGCCGGCGATATCGCT GCGGTGGCGAAAATGAAACAATATGAAGCGGTGCGGTCGGATGAAGCGGT CTATGGCAAAGGCGTCAAGCGGTCGCTGCCGGACGAACAGACGCTTGCTGA GCATCTCGTTCGCAAAGCGGCAGCCATTTGGGCGCTTGAGCAGCCGTTTATG GACGATTTGCGGAACAACGAACAAGATCAATTATTAACGAAGCTTGAGCAG CCGCTGGCGGCGATTTTGGCTGAAATGGAATTCACTGGGGTGAACGTGGAT ACAAAGCGGCTTGAACAGATGGGTTCGGAGCTCGCCGAACAACTGCGTGCC ATCGAGCAGCGCATTTACGAGCTAGCCGGCCAAGAGTTCAACATTAACTCAC CAAAACAGCTCGGAGTCATTTTATTTGAAAAGCTGCAGCTACCGGTGCTGAA GAAGACGAAAACAGGCTATTCGACTTCGGCTGATGTGCTTGAGAAGCTTGC GCCGCATCATGAAATCGTCGAAAACATTTTGCATTACCGCCAGCTTGGCAAA CTGCAATCAACGTATATTGAAGGATTGTTGAAAGTTGTGCGCCCTGATACCG GCAAAGTGCATACGATGTTCAACCAAGCGCTGACGCAAACTGGGCGGCTCA GCTCGGCCGAGCCGAACTTGCAAAACATTCCGATTCGGCTCGAAGAGGGGC GGAAAATCCGCCAAGCGTTCGTCCCGTCAGAGCCGGACTGGCTCATTTTCGC CGCCGATTACTCACAAATTGAATTGCGCGTCCTCGCCCATATCGCCGATGAC GACAATCTAATTGAAGCGTTCCAACGCGATTTGGATATTCACACAAAAACGG CGATGGACATTTTCCATGTGAGCGAAGAGGAAGTCACGGCCAACATGCGCC GCCAGGCAAAGGCCGTTAACTTCGGTATCGTTTACGGAATTAGCGATTACG GATTGGCGCAAAACTTGAACATTACGCGCAAAGAAGCTGCCGAATTTATCG AACGTTACTTCGCCAGCTTTCCGGGCGTAAAGCAGTATATGGAAAACATTGT GCAAGAAGCGAAACAGAAAGGATATGTGACAACGCTGTTGCATCGGCGCC GCTATTTGCCTGATATTACAAGCCGCAATTTCAACGTCCGCAGTTTTGCAGA GCGGACGGCCATGAACACGCCAATTCAAGGAAGCGCCGCTGACATTATTAA AAAAGCGATGATTGATTTAGCGGCACGGCTGAAAGAAGAGCAGCTTCAGGC TCGTCTTTTGCTGCAAGTGCATGACGAGCTCATTTTGGAAGCGCCAAAAGAG GAAATTGAGCGATTATGTGAGCTTGTTCCGGAAGTGATGGAGCAGGCCGTT |
| | ACGCTCCGCGTGCCGCTGAAAGTCGACTACCATTACGGCCCAACATGGTATG ATGCCAAATAA |
| SEQ ID NO: 17 is the amino acid sequence of the Bst DNA Polymerase, large fragment | AEGEKPLEEMEFAIVDVITEEMLADKAALVVEVMEENYHDAPIVGIALVNEHGR FFMRPETALADSQFLAWLADETKKKSMFDAKRAVVALKWKGIELRGVAFDLLL AAYLLNPAQDAGDIAAVAKMKQYEAVRSDEAVYGKGVKRSLPDEQTLAEHLVR KAAAIWALEQPFMDDLRNNEQDQLLTKLEQPLAAILAEMEFTGVNVDTKRLEQ MGSELAEQLRAIEQRIYELAGQEFNINSPKQLGVILFEKLQLPVLKKTKTGYSTSA DVLEKLAPHHEIVENILHYRQLGKLQSTYIEGLLKVVRPDTGKVHTMFNQALTQT GRLSSAEPNLQNIPIRLEEGRKIRQAFVPSEPDWLIFAADYSQIELRVLAHIADDD NLIEAFQRDLDIHTKTAMDIFHVSEEEVTANMRRQAKAVNFGIVYGISDYGLAQ NLNITRKEAAEFIERYFASFPGVKQYMENIVQEAKQKGYVTTLLHRRRYLPDITSR NFNVRSFAERTAMNTPIQGSAADIIKKAMIDLAARLKEEQLQARLLLQVHDELIL EAPKEEIERLCELVPEVMEQAVTLRVPLKVDYHYGPTWYDAK |

### DETAILED DESCRIPTION OF THE INVENTION

The present invention relates to a method for nucleic acid amplification, and detection or sequencing, comprising the steps of,
a. providing a double-stranded target nucleic acid molecule for amplification,
b. terminally attaching to said target nucleic acid molecule both up-stream and down-stream a first and second linker nucleic acid comprising a protelomerase target sequence, preferably as encoded in SEQ ID NO. 15
c. reacting said target nucleic acid molecule with a protelomerase, preferably as encoded by SEQ ID NO.1, such that the target nucleic acid is converted to a circular target nucleic acid,
d. amplifying the circular target nucleic acid in an amplification reaction comprising a polymerase and at least one first primer which binds outside the protelomerase target sequence such that an in parts self-complimentary amplification product is synthesized.

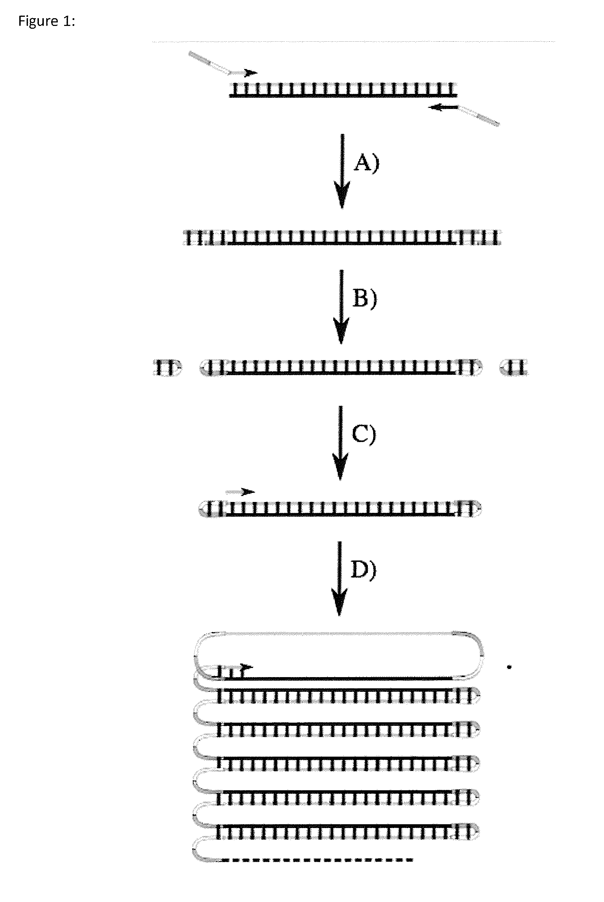

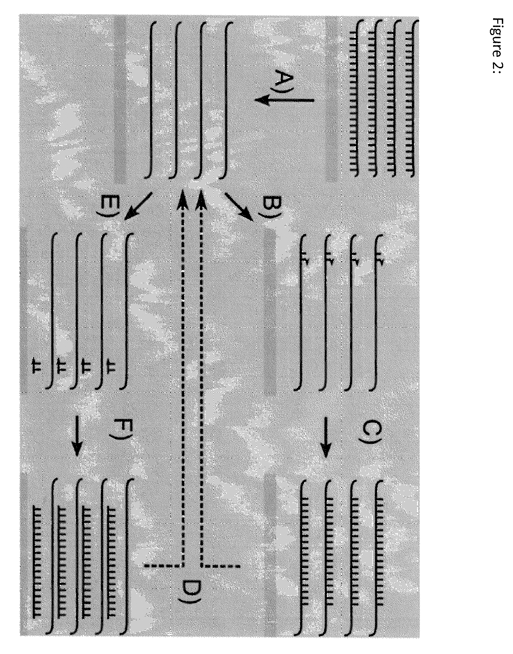

The invention astonishingly results in a single stranded nucleic acid molecule amplification product with a "snake like" structure; see figures 1 and 2. Here, the two originally complementary strands of the target nucleic acid molecule are now arranged in a concatemeric fashion. It may be assumed that these strands (now on one strand) bind each other to form said "snake-like" molecule. Due to the fact that the amplification product is one molecule per primer no free floating molecules come into existence, once the primer is bound to a solid phase after amplification. This makes such a "snake" nucleic acid the ideal template for detection or sequencing.

The double stranded target nucleic acid is preferably DNA. It may however, stem from an RNA molecule and be cDNA.

The step of terminally attaching to said target nucleic acid molecule both up-stream and down-stream a first and second linker nucleic acid comprising a protelomerase target sequence may be accomplished in numerous different ways. It is however, an essential step in so far as the protelomerase requires a specific template in order to accomplish the required circulization resulting in the closed linear DNA structure required for amplification.

In one embodiment of the invention the amplification product may for example be used for next generation sequencing. In such an embodiment it is preferred if the product is then attached to a solid phase.

Preferably, the amplification product is detected or sequenced.

The present invention preferably involves the step of sequencing the amplification product. The current method has the advantage that it is not restricted to a particular sequencing method. However, a next generation sequencing method is preferred. It is an advantage of the present method, that the product may be sequenced from both ends.

DNA sequencing techniques are of major importance in a wide variety of fields ranging from basic research to clinical diagnosis. The results available from such technologies can include information of varying degrees of specificity. For example, useful information can consist of determining whether a particular polynucleotide differs in sequence from a reference polynucleotide, confirming the presence of a particular polynucleotide sequence in a sample, determining partial sequence information such as the identity of one or more nucleotides within a polynucleotide, determining the identity and order of nucleotides within a polynucleotide, etc.

The sequencing step is preferably done by means of next generation sequencing. Manufacturers and technologies are Solexa/lllumina which generate up to 600 Gigabase (Gb) of 36 or 150 bp, Roche/454 which generate up to 700 Mbp reads of 400-1000 bp, ABI/SOLiD™ which generate > 20 Gb/day reads of 35-75 bp, Helicos which generate 21 - 35 Gb reads of 25-45 bp and Complete Genomics (a service company). Other manufacturers include Pacific Bioscience commercializing PacBio RS.

The Solexa/Illumina sequencing by synthesis technology is based on reversible dye-terminators. DNA molecules are first attached to primers on a slide and amplified so that local clonal colonies are formed (bridge amplification). Four types of reversible terminator bases (RT-bases) are added, and non-incorporated nucleotides are washed away. Unlike pyrosequencing, the DNA can only be extended one nucleotide at a time. A camera takes images of the fluorescently labelled nucleotides, then the dye along with the terminal 3' blocker is chemically removed from the DNA, allowing the next cycle (Brenner et al., Nature Biotechnol. 2000. 18(6):630-634).

The SOLiD™ ("Sequencing by Oligonucleotide Ligation and Detection") method (Life Technologies; WO 06/084132 A2) is based on the attachment of PCR amplified fragments of template nucleic acids via universal adapter sequences to magnetic beads and subsequent detection of the fragment sequences via ligation of labelled probes to primers hybridized to the adapter sequences. For the read-out a set of four fluorescently labelled di-base probes are used. After read-out, parts of the probes are cleaved and new cycles of ligation, detection and cleavage are performed. Due to the use of di-base probes, two rounds of sequencing have to be performed for each template sequence. Other methods and devices include Ion Torrent and Roche's 454.

PacBio RS is a single molecule real-time sequencing (SMRT) platform based on the properties of zero-mode waveguides (ZMW). A single DNA polymerase enzyme is affixed at the bottom of a ZMW with a single molecule of DNA as a template. The ZMW is a structure that creates an illuminated observation volume that is small enough to observe only a single nucleotide of DNA being incorporated by DNA polymerase. Each of the four DNA nucleotides is attached to one of four different fluorescent dyes. When a nucleotide is incorporated by the DNA polymerase, the fluorescent tag is cleaved off and diffuses out of the observation area of the ZMW where its fluorescence is no longer observable. A detector detects the fluorescent signal of the nucleotide incorporation, and the base call is made according to the corresponding fluorescence of the dye.

GridION and MinION systems developed by Oxford Nanopore Technologies use biological nanopores to sequence single molecules in a so-called DNA strand sequencing approach. A mutant variant of the pore protein, alpha-hemolysin, is inserted in a synthetic proprietary polymer bilayer that restricts ion passage to the pores alone. ONT uses a special enzyme to process the DNA, not the DNA polymerases currently used by some academic teams. The enzyme is capable of ratcheting DNA through the pore at upwards of 1,000 bases/second, a rate that is controlled by various cofactors in solution. The sequence is read by monitoring an electrical signal as a strand of DNA passes through the pore protein. An informatics system reads overlapping nucleotide triplets in the middle of the pore to deduce the sequence. By engineering a hairpin at the end of the molecule, both strands of the template DNA can be sequenced in succession on the same pore. This redundancy is used to reduce the error rate to an acclaimed 4%. Also these sequencing methods are claimed in the method herein.

The current method has the advantage that it is not restricted to a particular sequencing method. If the sequencing step is done by next generation sequencing, it is preferred that the method applied is selected from the group of those described above.

The amplification product may additionally be detected and/or quantified.

The detection step may be done by incorporating into the amplification product detectable probes, e.g. fluorescently labelled probes. A probe according to the present invention is an oligonucleotide, nucleic acid or a fragment thereof, which is substantially complementary to a specific nucleic acid sequence. Suitable hybridization probes include the LightCycler probe (Roche), the TaqMan probe (Life Technologies), a molecular beacon probe, a Scorpion primer, a Sunrise primer, a LUX primer and an Amplifluor primer.

The detection step may be alternatively done by using double-stranded DNA-binding dyes (e.g. SYBR Green) as reporters in a real-time PCR. A DNA-binding dye binds to all double-stranded DNA in PCR, causing fluorescence of the dye. An increase in DNA product during PCR therefore leads to an increase in fluorescence intensity and is measured at each cycle, thus allowing DNA concentrations to be quantified.

The quantification step may be based on quantitative real-time PCR using the techniques described before.

Preferably, the attachment of the first and second linker nucleic acids is performed with a reaction selected from the group of transposase reaction, recombinase reaction, ligase reaction and extension PCR.

Ideally, the amplification step is performed in an isothermal reaction and the polymerase used is preferably a phi29 polymerase.

In general the DNA template is contacted with at least one DNA polymerase under conditions promoting amplification of said template. Any DNA polymerase may be used in a process for amplification of closed linear DNA of the invention. Any commercially available DNA polymerase is suitable for use in this process of the invention. Two, three, four, five or more different DNA polymerases may be used, for example one which provides a proofreading function and one or more others which do not.

DNA polymerases having different mechanisms may be used e.g. strand displacement type polymerases and DNA polymerases replicating DNA by other methods. A suitable example of a DNA polymerase that does not have strand displacement activity is T4 DNA polymerase.

It is preferred that a DNA polymerase is highly stable, such that its activity is not substantially reduced by prolonged incubation under process conditions.

Therefore, the enzyme preferably has a long half-life under a range of process conditions including but not limited to temperature and pH. It is also preferred that a DNA polymerase has one or more characteristics suitable for a manufacturing process.

The DNA polymerase preferably has high fidelity, for example through having proof-reading activity. Furthermore, it is preferred that a DNA polymerase displays high processivity, high strand-displacement activity and a low Km for dNTPs and DNA. It is preferred that a DNA polymerase does not display non-specific exonuclease activity.

The skilled person can determine whether or not a given DNA polymerase displays characteristics as defined above by comparison with the properties displayed by commercially available DNA polymerases, e.g phi29, DeepVent^{®} and Bacillus stearothermophilus (Bst) DNA polymerase I, (see respective sequences above) respectively.

Bst DNA polymerase I is commercially available from New England Biolabs, Inc. Where a high processivity is referred to, this typically denotes the average number of nucleotides added by a DNA polymerase enzyme per association/dissociation with the template, i.e the length of primer extension obtained from a single association event.

Strand displacement-type polymerases are preferred for use in a process for amplification of closed linear DNA of the invention.

Preferred strand displacement-type polymerases are Phi 29 (SEQ ID NO: 2), Deep Vent^{®} and Bst DNA polymerase I (SEQ ID NO: 16) or variants of any thereof. Variants of these may be as defined below in relation to protelomerase enzymes. The term "strand displacement" is used herein to describe the ability of a DNA polymerase to displace complementary strands on encountering a region of double stranded DNA during DNA synthesis. Claimed herein are all those enzymes as shown in the sequence alignment. Obviously the polymerases for the amplification step and the protelomerases for the circulization step.

It should be understood that strand displacement amplification methods differ from PCR-based methods in that cycles of denaturation are not essential for efficient DNA amplification, as double-stranded DNA is not an obstacle to continued synthesis of new DNA strands.

In contrast, PCR methods require a denaturation step (i.e. elevating temperature to 94 degrees centigrade or above) in each cycle of the amplification process to melt double-stranded DNA and provide new single stranded templates.

A strand displacement DNA polymerase used in a process of the invention preferably has a processivity (primer extension length) of at least 20 kb, more preferably, at least 30 kb, at least 50 kb, or at least 70 kb or greater. In particularly preferred embodiments, the strand displacement DNA polymerase has a processivity that is comparable to, or greater than phi29 DNA polymerase.

A preferred strand displacement replication process is rolling circle amplification (RCA). The term RCA describes the ability of RCA-type DNA polymerases (also referred to herein as RCA polymerases) to continuously progress around a circular DNA template strand whilst extending a hybridised primer. This leads to formation of linear single stranded products with multiple repeats of amplified DNA.

RCA polymerases are particularly preferred for use in a process of the present invention.

The contacting of the template with the polymerase and at least one primer takes place under conditions promoting annealing of primers to the DNA template.

The conditions include the presence of single-stranded DNA allowing for hybridisation of the primers.

The conditions also include a temperature and buffer allowing for annealing of the primer to the template.

Appropriate annealing/hybridisation conditions may be selected depending on the nature of the primer. An example of preferred annealing conditions used in the present invention include a buffer 30 mM Tris-HCl, pH 7.5, 20 mM KCI, 8 mM MgCl₂.

Phi29 conditions are ideally, 50 mM Tris-HCl, 10 mM (NH₄)₂SO₄, 10 mM MgCl₂, 4 mM Dithiothreitol, pH 7.5 at 25°C.

The annealing may be carried out following denaturation by highly controlled gradual cooling to the desired reaction temperature. Typical cooling rates in degrees centigrade per minute are 1.0 to 5.0 but preferably 0.1 to 1.0, 0.3 to 1.0, 0.5 to 1.0 or 0.7 to 1.0.

During cooling, the temperature may be held at specific temperatures within the cooling range for periods of 1 to 10 minutes to create an optimal temperature profile for the primer to template annealing process.

This is advantageous to allow maximum binding of the primer to the template before the template itself renatures.

Once the template is contacted with the polymerase and primer, there is then a step of incubation under conditions promoting amplification of said template. Preferably, the conditions promote amplification of said template by displacement of replicated strands through strand displacement replication of another strand.

The conditions comprise use of any temperature allowing for amplification of DNA, commonly in the range of 20 to 90 degrees centigrade. A preferred temperature range may be about 20 to about 40 or about 25 to about 35 degrees centigrade. For phi29 these temperatures are good. For Bst 55-70 degrees would be good.

Typically, an appropriate temperature is selected based on the temperature at which a specific polymerase has optimal activity.

For example, where phi29 DNA polymerase is used, a suitable temperature range would be about 25 to about 35 degrees centigrade, preferably about 30 degrees centigrade.

Other conditions promoting amplification of the DNA template comprise the presence of a DNA polymerase and one or more primers. The conditions also include the presence of all four dNTPs, ATP, TTP, CTP and GTP, suitable buffering agents/pH and other factors which are required for enzyme performance or stability. Suitable conditions include any conditions used to provide for activity of DNA polymerase enzymes known in the art.

For example, the pH may be within the range of 3 to 10, preferably 5 to 8 or about 7, such as about 7.5. pH may be maintained in this range by use of one or more buffering agents.

Such buffers include, but are not restricted to MES, Bis-Tris, ADA, ACES, PIPES, MOBS, MOPS, MOPSO, Bis-Tris Propane, BES, TES, HEPES, DIPSO, TAPSO, Trizma, HEPPSO, POPSO, TEA, EPPS, Tricine, Gly-Gly, Bicine, HEPBS, TAPS, AMPD, TABS, AMPSO, CHES, CAPSO, AMP, CAPS, CABS, phosphate, citric acid-sodium hydrogen phosphate, citric acid-sodium citrate, sodium acetate-acetic acid, imidazole and sodium carbonate-sodium bicarbonate.

The reaction may also comprise salts of divalent metals such as but not limited to salts of magnesium (Mg) and manganese (Mn), including chlorides, acetates and sulphates.

Salts of monovalent metals may also be included, such as sodium salts and potassium salts, for example potassium chloride. Other salts that may be included are ammonium salts, in particular ammonium sulphate.

Detergents may also be included. Examples of suitable detergents include Triton X-100, Tween 20 and derivatives of either thereof. Stabilising agents may also be included in the reaction. Any suitable stabilising agent may be used, in particular, bovine serum albumin (BSA) and other stabilising proteins. Reaction conditions may also be improved by adding agents that relax DNA and make template denaturation easier. Such agents include, for example, dimethyl sulphoxide (DMSO), formamide, glycerol and betaine.

It should be understood that the skilled person is able to modify and optimise amplification and incubation conditions for a process of the invention on the basis of their general knowledge. Likewise the specific concentrations of particular agents may be selected on the basis of previous examples in the art and further optimised on the basis of general knowledge. As an example, a suitable reaction buffer used in RCA-based methods in the art is 50 mM Tris HCl, pH 7.5, 10 mM MgCl₂, 20mM (NH4)₂SO₄, 5% glycerol, 0.2 mM BSA, 1 mM dNTPs. A preferred reaction buffer used in the RCA amplification of the invention is 35 mM Tris-HCl, 50 mM KCI, 14 mM MgCl₂, 10 mM (NH₄)₂SO4, 4mM DTT, 1 mM dNTP. This buffer is particularly suitable for use with phi29 RCA polymerase.

It is important that the amplification primer does not bind exclusively in the protelomerase binding site. Preferably also said one first primer in the amplification step d) is labeled and the label is selected from the group of biotin, thiol, or digoxygenin.

Preferably, the protelomerase enzyme is from Agrobacterium tumefaciens and is encoded by SEQ ID NO. 13.

In one embodiment of the invention the unbound amplification primer of step d) is eliminated prior to amplification in order to avoid false priming events. Various methods exist for doing this, using single-strand specific exonucleases, or a size exclusion purification step.

In order to prevent aggregation or unwanted cross-hybridisation of replicating nanoballs, the amplification process of single templates can occur in separate compartments. This can be performed by amplification in water-in-oil emulsions, liposomes, or embedded in resins or gels. After the amplification process, the compartments are broken down and the separately generated DNA nanoballs are purified for the following sequencing process.

Prior to sequencing the amplification product template created may be attached to a solid phase. This may be effected either by hybridization to a surface-immobilized amplification primer, or unspecific attachment such as hydrogen bonding, van der Waals forces, and the like.

### FIGURE CAPTIONS

### Figure 1:

Double-stranded linear DNA is A) tailed by extension PCR with end-specific primers that each add a protelomerase substrate sequence to the termini. B) Resulting PCR product is treated with protelomerase to generate a linear covalently-closed double-stranded DNA template with terminal hairpins. C) By addition of a specific primer, a rolling circle amplification is initiated, D) resulting in one snake-like self-complementary concatemer generated from one template.

### Figure 2:

The snake-like concatemer DNA is immobilised on a surface and A) denatured to largely dissociate self-complementary sequences. B) the denatured concatemer is incubated with one sequencing primer specific for the end of one strand stemming from the initial amplification template. C) the sequence of this strand is identified by a next-generation sequencing method. D) the denatured strand (A) is regenerated. E) the denatured concatemer is incubated with a different sequencing primer specific for the end of opposite strand stemming from the initial amplification template. F) the sequence of this strand is identified by a next-generation sequencing method. Finally, this product can be regenerated as in D) to allow additional rounds of sequencing.

## Claims

1. Method for nucleic acid amplification, and detection or sequencing, comprising the steps of,
a. providing a double-stranded target nucleic acid molecule for amplification,
b. terminally attaching to said target nucleic acid molecule both up-stream and down-stream a first and second linker nucleic acid comprising a protelomerase target sequence,
c. reacting said target nucleic acid molecule with a protelomerase, preferably as encoded by SEQ ID NO. 13, such that the target nucleic acid is converted to a circular target nucleic acid,
d. amplifying the circular target nucleic acid in an amplification reaction comprising a polymerase and at least one first primer which binds outside the protelomerase target sequence such that an in parts self-complimentary amplification product is synthesized.

2. Method according to claim 1, wherein after amplification the amplification product is attached to a solid phase or the amplification step takes place on a solid phase.

3. Method according to claims 1 or 2, wherein the attachment of the first and second linker nucleic acids is performed with a reaction selected from the group of, transposase reaction, recombinase reaction, ligase reaction and extension PCR.

4. Method according to claims 1 to 3, wherein the amplification step is performed in an isothermal reaction and the polymerase used is preferably a Phi29 polymerase.

5. Method according to claims 1 to 4, wherein the sequencing of the amplification product is done with a next generation sequencing method selected from the group of sequencing by synthesis with reverible terminators or single-molecule sequencing with nanopores.

6. Method according to claims 1 to 5, wherein said one first primer in the amplification step d) is labeled and the label is selected from the group of biotin, thiol, or digoxygenin.

7. Method according to claims 1 to 6, wherein the unbound amplification primer of step d) is eliminated prior to amplification in order to avoid false priming events.

8. Method according to claims 1 to 7, wherein the protelomerase enzyme is from *Agrobacterium tumefaciens* and is encoded by SEQ ID NO. 13.

9. Method according to claims 1 to 8, wherein the amplification product is detected or sequenced.

10. Kit for a method for nucleic acid amplification according to any of the claims 1 to 9, comprising a protelomerase enzyme as well as an amplification primer.

11. Use of the amplification product according to claims 1 to 8, as a template for sequencing.
